# EUROPÄISCHE PATENTANMELDUNG

(11) **EP 3 984 518 A1**
(43) Veröffentlichungstag der Anmeldung: **20.04.2022**
(21) Anmeldenummer: 21194809.6
(22) Anmeldetag: 03.09.2021
(51) Int. Cl.: A61K 8/02, A61K 8/20, A61K 8/92, A61K 8/96, A61Q 19/10, C02F 1/68, E03C 1/046

(54) **GEPRESSTER FORMKÖRPER SOWIE APPLIKATOR ZUR AUFNAHME EINES SOLCHEN FORMKÖRPERS**

(30) Priorität: 21.09.2020 DE 102020124503
(71) Anmelder: Showerplus Entwicklungs- und Vertriebsgesellschaft mbH, 31137 Hildesheim (DE)
(72) Erfinder: Sackel, Rene, 31141 Hildesheim (DE); Benedikt, Linne, 31137 Hildesheim (DE)
(74) Vertreter: Eisenführ Speiser

(57) **Zusammenfassung**

Die Erfindung betrifft ein Verfahren eines gepressten Formkörpers (1), der aus wasserlöslichen Salzpartikeln besteht, die eine vorbestimmte Partikelgröße aufweisen, wobei der Mehrzahl der gepressten Partikel eine Partikelgröße aufweist, die im Bereich von 0,2mm bis 3,5mm liegt, vorzugsweise im Bereich von 0,5mm bis 2,5mm liegt, wobei die Partikel mit einem vorbestimmten Pressdruck zur Ausbildung des Formkörpers verpresst sind, wobei der Formkörper eine vorbestimmte Form, Oberfläche und Querschnitt, ein vorbestimmtes Volumen und eine vorbestimmte Masse aufweist und innerhalb einer vorbestimmten Zeit, z.B. innerhalb von 1 bis 10 Minuten, vorzugsweise etwa 1 bis 5 Minuten durch eine vorbestimmte Mindestmenge Wasser, die innerhalb der vorbestimmten Zeit den Formkörper umfließt, die Partikel des Formkörpers zu einen vorbestimmten Prozentsatz, vorzugsweise mehr als 90%, besonders bevorzugt bis zu 100% auflöst.

## Beschreibung

Die vorliegende Erfindung betrifft einen festen, gepressten Formkörper sowie einen Applikator zur Aufnahme eines solchen Formkörpers. Der feste Formkörper besteht dabei aus mindestens einer ionischen Verbindung, zum Beispiel NaCl, und in mindestens einer Ausprägung kann der feste Formkörper auch unterschiedliche wasserlösliche oder -unlösliche Zusätze aufweisen.

Soweit in der vorliegenden Anmeldung auch feste Formkörper mit wasserlöslichen oder wasserunlöslichen festen Zusätzen versehen ist, können diese Zusätze Pflanzenteile sein, insbesondere auf eine bestimmte Größe zerkleinerte Pflanzenteile, z.B. Blätter, Blüten, Stängel oder dergleichen von Pflanzen, wie z.B. Lavendel, Rosenblüten, Holz, Nüssen aber es ist auch möglich, dass bestimmter Tee, z.B. Kräutertee mit Bestandteile (Blätter, Stängel, Blüten, etc.) in einer bestimmten Korngröße mit in dem Formkörper enthalten sind.

Ferner ist es gemäß der vorliegenden Erfindung aber auch möglich, dass der erfindungsgemäße feste Formkörper mit flüssigen Zusätzen versehen wird, z.B. Babyöl, Paraffinum liquidum, vaselinartige Substanzen, Fette Öle, Cocos Nucifera Oil (Kokosöl), Elaeis Guineensis Oli (Palmöl), Glycine Soja Oil (Sojaöl), Hydrogenated Olive Oil (Olivenöl), Persea Gratissima Oil) Avocadoöl), Prunus Amygdalus Dulcis Oil (Mandelöl), Shea Butter Glycerides (Sheabutter), Simondsia chinensis Oil (Jojobaöl). Diese flüssigen Zusätze werde bei Auflösung des Formkörpers durch Umspülung von Wasser ebenfalls mit aufgelöst und gelangen somit ins Duschwasser.

Für die Anwendung im Hygienebereich, z.B. einer Dusche umfasst dabei auch die Anwendung im Wellnessbereich, Sportbereich, Campingbereich aber auch Medizinbereich und dergleichen. Unter Wellness wird bevorzugt der Zustand des Wohlbefindens und/oder der Gesundheit verstanden und die Erfindung gemäß der vorliegenden Anmeldung dient insbesondere dazu, das Wohlbefinden und/oder die Gesundheit des Benutzers zu steigern.

Der gepresste Formkörper dient dabei zum Einsatz in einen Applikator, in dem die Bestandteile des Formkörpers bei Durchfluss bzw. Vorbeifluss, einem Bad oder dergleichen von Wasser am Formkörper vorbei aufgelöst werden und/oder mitgespült werden und so eine angereicherte wässrige Lösung für die Anwendung im Hygienebereich, zum Beispiel in einer Dusche bereitgestellt wird.

Soweit in der vorliegenden Anmeldung einen Applikator genannt und beschrieben wird, ist dies eine Einrichtung, die dazu geeignet ist, einen Formkörper aufzunehmen. Dieser Applikator kann dabei in einer Badinfrastruktur ganz unterschiedlich eingesetzt werden, z.B. zwischen einer Mischbatterie und einem Duschkopf eingebaut sein. Es ist auch möglich, dass der Applikator als Teil einer Kombination, also eines Systems ist aus einer Mischbatterie und dem Applikator zur Aufnahme des Formkörpers besteht. In diesem Fall ist der Applikator dann nicht zerstörungsfrei von Mischbatterie zu lösen, sondern Mischbatterie und Applikator bilden ein Teil. Weiterhin ist es möglich, dass der Applikator ein Teil bildet, welches den Teil einer Kombination mit einem Duschkopf bildet, sodass dann Applikator und Duschkopf mittels eines Werkzeugs nicht zerstörungsfrei voneinander trennbar sind. Es ist auch möglich, dass der Applikator den Formkörper mit weiteren Zusätzen, Additiven, z.B. ätherischen Ölen, etc. aufnehmen kann. Es ist aber auch möglich, dass der Applikator statt des Formkörpers Kräuter, Pflanzen, eine Creme oder andere Stoffe aufnimmt, die bei Umspülung mit Wasser Inhaltsstoffe abgeben und/oder sich sogar vollständig im Wasser auflösen, z.B. bei einer wasserlöslichen Creme.

Mit Hilfe des hier beschriebenen Formkörpers ist es möglich, mit Ionen und weiteren Zusätzen angereichertes Wasser, beispielsweise für eine Körperdusche, bereitzustellen, die als Wellnessdusche oder in einer speziellen Ausprägung zur Linderung von Symptomen von Hautkrankheiten bzw. -beeinträchtigungen eingesetzt werden kann. Auch kann eine solche Körperdusche bevorzugt von Menschen eingesetzt werden, die unter verschiedensten Symptomen einer Neurodermitis oder Hautbeeinträchtigungen, zum Beispiel Juckreiz, trockene Haut etc. leiden. Eine Wellnessdusche ist nach dem Verständnis der vorliegenden Erfindung dabei eine Duscheinrichtung, die dem gesteigerten Wohlbefinden des Nutzers dienst.

Durch die Erfindung der vorliegenden Anmeldung wird mithin eine Beauty-Tec-Einrichtung geschaffen, die je nach Verwendung des Inhaltsstoffs, z.B. im gepressten Formkörper oder separat hiervon, sowohl eine hautpflegende Behandlung ermöglicht aber durch Freisetzung von Inhaltsstoffen, z.B. Aromen, Geruchstoffen, etc. auch das Wohlbefinden in der Dusche durch Einatmen angenehmer Düfte steigert.

Ein Applikator, also eine Apparatur zur Anreicherung von Flüssigkeitsströmen im Hygienebereich ist unter anderem offenbart in DE 197 02 315. Ein Nachteil des Applikators liegt in der Zuführung eines flüssigen Mediums, welches eine portionierte Dosierung durch den Anwender erschwert.

Ferner ist in DE 20 2008 013 784 ein Applikator beschrieben, welcher die Zuführung eines Zusatzstoffes in einen Duschkopf beschreibt. Dabei wird eine lose Materialschüttung beschrieben, welche in den Duschkopf eingeführt bzw. eingebracht werden kann. Auch dieses Vorgehen erschwert eine genau abgemessene Dosierung und gestaltet den Nachfüllprozess für die Materialschüttung für den Anwender sehr umständlich.

In US 2017/233273 und US 2019/314807 ist ein durch Pressen erzeugter Salzkörper zum Einsatz in einem lonentauscher beschrieben. Der bevorzugte Zweck besteht dabei darin, "hartes Wasser" aufzuweichen. Der aus der vorgenannten Schrift bekannte Salzkörper ist dabei so beschaffen, dass es sehr lange braucht, um den Körper selbst aufzulösen, um somit möglichst lange den gewünschten Effekt der Aufweichung harten Wassers bereitstellen zu können.

In WO 00/73208 A1 sind gepresste Formen aus Natriumchlorid mit möglichen Zusätzen unter Verwendung von Ammonium Hydroxy-poly-carboxylsäuren als anti-Verklumpungsmittel beschrieben. Es werden dabei unterschiedliche Formen wie Ziegel, Stäbe, Presslinge usw. beschrieben. Eine Anwendung der gepressten Salzkörper für den Hygienebereich ist völlig ungeeignet und beim möglichen Augenkontakt sogar für den Benutzer gefährlich.

In WO 2018/178980 ist die Herstellung von gepressten Formkörpern aus Salz beschrieben, die im Wesentlichen als Nahrungsmittelzusätze, insbesondere in der Vieh- und Tierhaltung Anwendung finden sollen.

Aus EP 1 008 554 A1 ist eine agglomerierte Salzpastille in zylindrischer Form mit einer gewölbten Seite bekannt, die ein vorbestimmtes Verhältnis von h/d (Höhe zu Durchmesser) zwischen 0,040 und 0,155 beschreibt.

In GB 2188915 wird eine Kompaktierung von Salz beschrieben, wobei das Partikelförmige Salz durch Befeuchten und anschließendes Trocknen in eine Form gebracht wird, um ein vorzeitiges Auseinanderbrechen verhindert.

In FR 2046692 ist ein gepresstes Salz in Form von Zylindern mit konvexen Enden beschrieben, die ein vorbestimmtes Verhältnis von h/d (Höhe zu Durchmesser) von 0.1 - 1.5 beschreiben.

In DE 35 16 606 werden Natriumchlorid-Kugeln mit einem Durchmesser von 0,5 mm bis 50 mm beschrieben. Diese Kugeln sollen als Regenerant für Kationen-Austauschmaterial zur Wasserenthärtung dienen.

Als weiterer Stand der Technik sind die Druckschriften DE 699 08 891 T2, GB 2 105 312 A, EP 3 339 517 A2, DE 20 2009 016 864 U1, US 2016/0129464 A1 und DE 600 10 254 T2 relevant.

Nachstehend beschrieben wird erfindungsgemäß ein System vorgeschlagen, welches aus einem Festkörper (nachfolgend auch Salzstick genannt) besteht, wobei Salz ein wesentlicher Bestandteil des Formkörpers ist und einem Applikator zur Aufnahme des festen Formkörpers, wobei der Applikator Teil eines Sanitärsystems, zum Beispiel einer Dusche, eines Bads oder dergleichen ist und bei der Wasserabgabe des Sanitärsystems der Applikator so eingestellt werden kann, dass das Wasser, welches das Sanitärsystem verlassen soll, z.B. die Duschabgabe zuvor den festen Formkörper umspült.

Der erfindungsgemäße gepresste Formkörper mit dem Merkmal nach Anspruch 1 besteht aus wasserlöslichen Salzpartikeln, die eine vorbestimmte Partikelgröße aufweisen, wobei der Mehrzahl der gepressten Partikel eine Partikelgröße aufweist, die im Bereich von 0,2mm bis 3,5mm liegt, vorzugsweise im Bereich von 0,5mm bis 2,5mm liegt, wobei die Partikel mit einem vorbestimmten Pressdruck zur Ausbildung des Formkörpers verpresst sind, wobei der Formkörper eine vorbestimmte Form, Oberfläche und Querschnitt, ein vorbestimmtes Volumen und eine vorbestimmte Masse aufweist und innerhalb einer vorbestimmten Zeit, z.B. innerhalb von 1 bis 10 Minuten, vorzugsweise etwa 1 bis 5 Minuten durch eine vorbestimmte Mindestmenge Wasser, die innerhalb der vorbestimmten Zeit den Formkörper umfließt, die Partikel des Formkörpers zu einen vorbestimmten Prozentsatz, vorzugsweise mehr als 90%, besonders bevorzugt bis zu 100% auflöst.

Wie nachstehend noch aufgezeigt, bestimmt u.a. die Form des festen Formkörpers aus Salz maßgeblich das Verhältnis von Volumen zu seiner Oberfläche und damit auch das Auflösungsverhalten des gesamten Formkörpers im Wasser bzw. bei Umströmung mit Wasser. Des Weiteren ist die Form des Formkörpers u.a. ausschlaggebend für die Einführung des Formkörpers in einen Applikator, da aufgrund eines Schlüssel-Schloss Prinzips nur geeignete Formkörper in den Applikator eingebracht werden können, um somit eine fälschliche Bedienung einerseits zu behindern und andererseits für die gewünschte Auflösung des Formkörpers bei der Wasserumspülung innerhalb einer vorbestimmten Zeit Sorge zu tragen.

Der Formkörper wird dabei in eine Kammer eines geeigneten Applikators eingebracht und im Durchfluss von Wasser innerhalb einer vorbestimmten Zeit zum Beispiel 1 - 5 Minuten aufgelöst. Hierbei stellt sich in Abhängigkeit der Durchflussrate (l/min) des Wassers, der Temperatur des Wassers und der Form des Formkörpers eine definierte lonenkonzentration im Wasser ein. In einer speziellen Ausprägung, in der sich wasserunlösliche Zusätze befinden, werden diese im Lösungsprozess des Formkörpers freigegeben und vom Wasser mitgespült. Somit ergibt sich eine ionenhaltige, wässrige Lösung, die in einer speziellen Ausprägung weitere wasserlösliche oder-unlösliche Zusätze enthält, die beispielsweise für eine Körperdusche bevorzugt eingesetzt werden können.

Als geeigneter Formkörper dienen dreidimensionale Formen, die in einer Dimension (x = Länge) mindestens das dreifache Ausmaß der jeweils beiden anderen Dimensionen (y = Breite, z = Tiefe/Höhe) aufweisen. Wird der Formkörper in der y-z Fläche (Querschnitt) betrachtet, weist er eine spezifische Grundform wie ein Kreis, ein Dreieck, ein Viereck oder komplexere Formen wie ein Pluszeichen oder ein abgerundetes Pluszeichen auf.

Diese definierten Formen erlauben es, nur geeignete Formkörper in den entsprechenden Applikator einzubringen, da der Formkörper in einer speziellen Ausprägung mit der y-z Fläche in eine Aufnahmeöffnung des Applikators eingebracht werden kann. Um eine einfache Einführung des Formkörpers in den Applikator zu erlauben, ist in dery-z Fläche mindestens eine zweizahnige Rotationsachse bzw. eine orthogonal zur y-z Fläche stehende Spiegelebene notwendig. Je nach Ausprägung sind mehrzahnige Rotationsachsen oder Spiegelebenen möglich.

Bei einer Ausführungsform der Erfindung ist es auch möglich, dass es zu einem bestimmten Prozentsatz, vorzugsweise bis zum 10%, vorzugsweise weniger als 5%, besonders bevorzugt 2% oder weniger, additiv den Partikeln, den insbesondere Salzpartikeln des Formkörpers beigemischt sind, vorzugsweise Zusätze, die eine körnige Struktur aufweisen, die vorzugsweise in der Größenordnung (oder geringer) der Partikel des Formkörpers liegen und/oder die ätherische Öle bilden und/oder aus Pflanzenmaterial bestehen, z.B. Blätter, Stängel, Blüten oder dergleichen von Kräutern, Pflanzen, insbesondere von solchen Kräuter, Pflanzen mit einer (nachgewiesenen) nachgemessenen Pflege- und Gesundheitsfunktion.

Eine Erweiterung der Ausführungsform ist das eingesetzte Additiv, z.B. ein ätherisches Öl, welches vor Verpressung mit den Partikeln (insbesondere Salzpartikeln, des Formkörpers vermischt wird und die Partikel im Formkörper bindet. Dabei ist es auch möglich, dass ein Additiv vor oder nach Verpressung des Formkörpers mit diesem vermischt wird bzw. diesem Formkörper zugesetzt wird.

Als ein solches Additiv bieten sich ätherische Öle an, z.B. auf Mineralölbasis (z.B. Babyöl, Paraffinum liquidum, vaselinartige Substanzen) oder auch Fette Öle, wie z.B. Cocos Lucifera Oil (Kokosöl), Elaeis Guineensis Oil (Palmöl), Glycine Soja Oil (Sojaöl), Hydrogenated Olive Oil (Olivenöl), Persea Gratissima Oil (Avocadoöl), Prunus Amygdalus Dulcis Oil (Mandelöl), Shea Butter Glycerides (Sheabutter), Simmondsia chinensis Oil (Jojobaöl) oder ein Tensid und/oder Lipid oder eine Fettphase hiervon oder dergleichen. Ein Additiv kann aber auch ein Tensid und/oder Lipid (oder Fettphase hiervon) sein, welches, wie erwähnt, nach dem Verpressen der Partikel zum Formkörper diesem zum Körper zugefügt wird, z.B. durch Tränken des Formkörpers in dem Additiv, also Öl, Tensid, etc.

Die Aufgabe der Erfindung besteht also darin, ein System und einen Formkörper auszubilden, der in einer vorbestimmten Zeit mit einer vorbestimmten Menge an Wasser aufgelöst werden kann und darüber hinaus soll auch ein entsprechender Applikator zur Verfügung gestellt werden, der die einfache und sichere Aufnahme für gewünschte Auflösung des Formkörpers und die Abgabe von Wasser z.B. beim Duschen mit einer gewünschten Salzkonzentration ermöglicht.

Die Aufgabe wird mit einem gepressten Formkörper mit den Merkmalen nach Anspruch 1 gelöst. Vorteile Weiterbildungen sind in den Ansprüchen 2 bis 8 beschrieben.

Anspruch 9 beschreibt einen erfindungsgemäßen Applikator mit einer Aufnahmekammer zur Aufnahme des Formkörpers und die Unteransprüche 10 bis 18 zeigen erfindungsgemäße Weiterbildungen des Applikators.

Die Erfindung wird nachstehend anhand eines Ausführungsbeispiels näher beschrieben.
- Figur 1a) bis e): zeigt eine fünf verschiedene Beispiele eines erfindungsgemäßen Formkörpers,
- Figur 2a) bis e): zeigt die entsprechenden Formkörper gemäß Fig. 1 aus der Frontperspektive wiederum mit den Abmaßen,
- Figur 3: zeigt eine Prinzipdarstellung eines erfindungsgemäßen Applikators, und
- Figur 4, 5 und 6: zeigen verschiedene Ansichten des erfindungsgemäßen Applikators mit einem darin untergebrachten erfindungsgemäßen Formkörper.

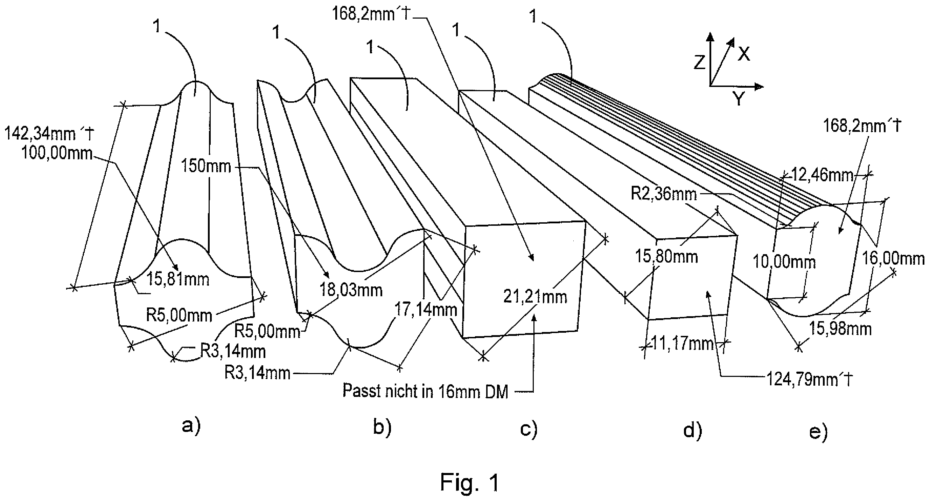

Dabei ist zu erkennen, dass die jeweiligen Formkörper 1 nach Figur 1a), b), c), d) und e) einen unterschiedlichen Querschnitt aufweisen, aber die gleiche Länge aufweisen. Besonders geeignet sind die Formkörper nach Figur 1a) und Figur 1b), eventuell auch nach Figur 1e), denn bei diesen Formkörpern ist das Oberflächen zu Volumen Verhältnis so gestaltet, dass es größer ist als das Oberflächen Volumen Verhältnis eines Quaders, Fig. 1c und d.

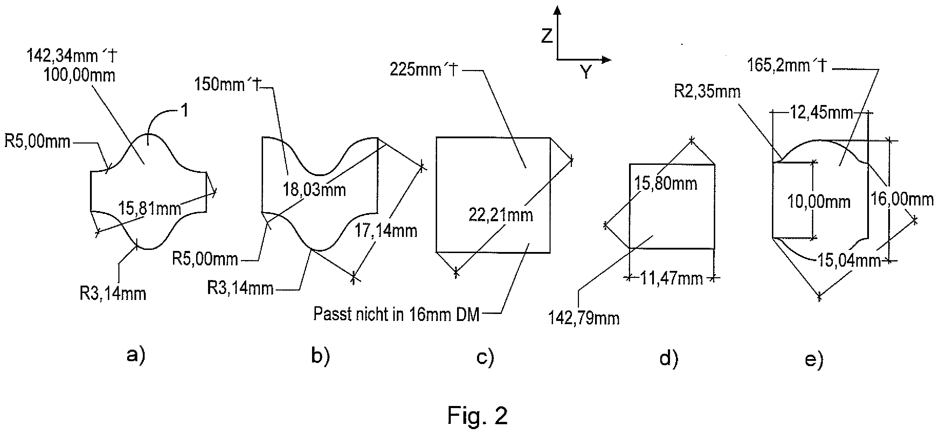

Die einzelnen Abmaße in der Figur 1 sind nur beispielhaft zu verstehen, um Überlegungen zu den Abmaßen des erfindungsgemäßen Formkörpers zu verdeutlichen. Figur 2a) bis e) zeigt die entsprechenden Formkörper 1 gemäß Figur 1 aus der Frontperspektive wiederum mit den beispielhaften Abmaßen.

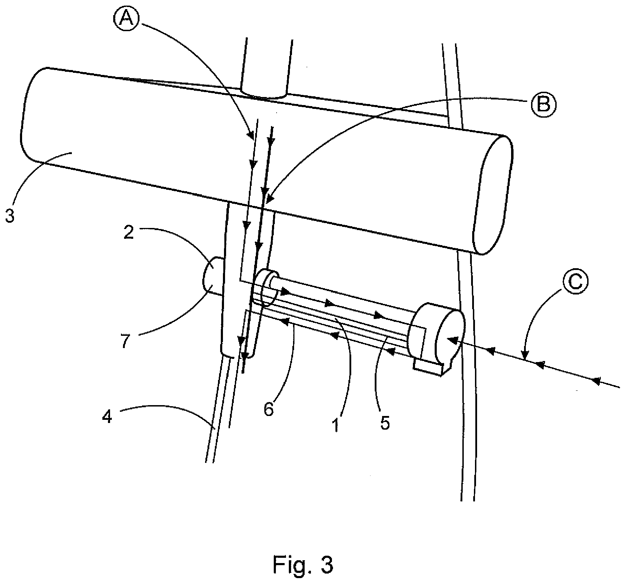

Figur 3 zeigt eine Prinzipdarstellung eines erfindungsgemäßen Applikators2, an welcher eine Standartsanitärarmatur 3 angebracht ist. Diese Standartsanitärarmatur erlaubt es beispielsweise dem Benutzer, eine vorbestimmte Temperatur des durchfließenden Wassers einzustellen, wie auch die Wassermenge.

In Figur 3 ist mit Bezugszeichen A auf den Durchflussverlauf bei Durchströmung von Wasser durch die mit dem Formkörper gefüllte Kammer 5 hingewiesen. Bezugszeichen B 2 zeigt den Durchflussverlauf des Wassers bei einem normalen Duschvorgang, also ohne Umspülung des Formkörpers 1, wobei dann das Wasser der Standartsanitärarmatur 3 den Applikator 2 durchfließt und z.B. über einen üblichen angeschlossenen Schlauch 4 zum Duschkopf gelangt, der am Schlauch 4 angeschlossen ist. Der Duschkopf ist im Bild nicht dargestellt.

Bezugszeichen C weist auf die Befüllungsrichtung hin, also die Richtung, mit welcher der feste Formkörper 1 in die Kammer 5 des Applikators 2 eingebracht wird. Nach dem Einbringen des festen Formkörpers 1 in die Kammer 5 des Applikators 2 wird die Kammer mit einem entsprechenden Abschluss, z.B. einer Muffe, einer Schraube oder dergleichen, verschlossen. In Figur 3 ist auch ein Rücklaufkanal 6 zu erkennen, der mi Weiteren noch näher erläutert wird. Dieser Rücklaufkanal 6 nimmt das Wasser auf, welches in die Kammer 5 des Applikators strömt und von dort dann in den Schlauch 4 fließt.

Am Applikator 2 ist ferner ein Bedienhebel 7 angebracht, mittels dem der Benutzer Einstellungen vornehmen kann, ob Wasser den Applikator längs des Durchflusspfades A, des Durchflusspfades B durchfließt oder u.U. sogar Wasser entlang beider Durchflusspfade A und B fließt.

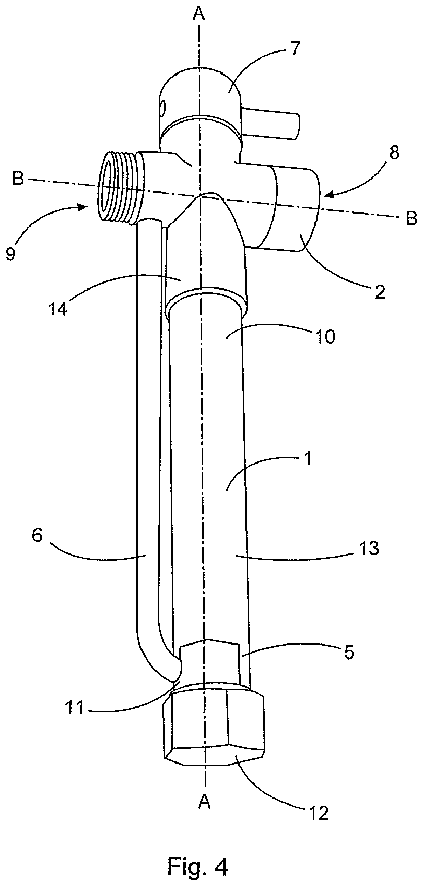

Figur 4, 5 und 6 zeigen verschiedene Ansichten des erfindungsgemäßen Applikators mit einem darin untergebrachten erfindungsgemäßen Formkörper.

Schließlich zeigen die Produktdatenblätter I, II, III und IV verschiedene Produktspezifikationen von verschiedenen Salzsorten, nämlich Kristallsalz aus Pakistan (Produktdatenblatt I), Alpensalz aus Österreich (Produktdatenblatt II), Deutsches Steinsalz (Produktdatenblatt III) sowie Kubisches Halitsalz (Produktdatenblatt IV).

Die Datenblätter zeigen dabei einerseits in welchen Körnungen die jeweiligen Produkte zu erhalten sind und beschreiben darüber hinaus die Herkunft, Farbe als auch die Zusammensetzung der jeweiligen Salze.

Zur Herstellung des erfindungsgemäßen Formkörpers 1 werden mithin Salzpartikel verwendet, die eine vorbestimmte Partikelgröße aufweisen, die bevorzugt im Bereich von 0,2 bis 3,5 mm pro Partikel liegt, vorzugsweise im Bereich von 0,5 bis 2,5 mm (Durchmesser) liegt. Sodann wird eine vorbestimmte Menge der Salzpartikel mit einem vorbestimmten Pressdruck zur Ausbildung des Formkörpers verpresst, wobei dann der gepresste Formkörper eine vorbestimmte Form, nämlich Länge, Oberfläche und Querschnitt und somit auch ein vorbestimmtes Volumen und eine vorbestimmte Masse aufweist.

Der Pressdruck sollte dabei möglichst größer sein als 10 KN/cm², vorzugsweise bei ca. 18 KN/cm² liegen, aber kleiner als der vorbestimmte Höchstpressdruck, zum Beispiel kleiner als 50 KN/cm², vorzugsweise kleiner als 25 KN/cm².

Durch den vorbeschriebenen Bereich des Pressdrucks weist der vorbestimmte Werkstoff, z. B. das Salz und / oder die darin enthaltenen Additive, während des Pressvorgangs ein Fließverhalten auf, welches durch das Wandern von Versetzungen in der Salzkristallstruktur erfolgt. Das Wandern von Versetzungen erfolgt hierbei entlang der fünf unabhängigen Gleitebenen (der Salzkristalle). Dieses besondere Verhalten des Mediums findet bis 10 KN/cm² gering statt, ab 15 KN/cm² ausreichend und ab 18 KN/cm² nicht mehr statt. Der Pressvorgang ist dadurch gekennzeichnet, dass eine plastische Verformung der Salzkristalle, wobei der entstehende Druck durch die vorbestimmte Pressform nach innen weicht, eine Dauerfestigkeit des Formkörpers sicherstellt. Die Herstellungsart hierfür ist z. B. ein Fließpressverfahren, welches folgende Fertigungssituation aufweisen sollte: Vorzugsweise ein Vorwärtsfließpressen in Verbindung mit Voll-Fließpressen, Hohl-Fließpressen als Kaltumformung.

Mit einem Vorwärtsfließpressen versteht man, dass der Werkstofffluss und die Stempelbewegungsrichtung gleich sind. Unter einem Rückwärtsfließpressen versteht man, dass der Werkstofffluss und die Stempelbewegung entgegengesetzt sind. Unter einem Querfließpressen versteht man, dass der Werkstofffluss quer zur Stempelbewegungsrichtung ist, Unter einem Vollfließpressen versteht man, dass der Querschnitt des Rohteils des Werkstoffs nicht durch Aussparung oder Formkern oder Dorn unterbrochen wird, unter einem Hohlfließpressen versteht man, dass der Querschnitt des Rohteils durch eine Aussparung oder Formkern oder Dorn unterbrochen wird, unter einer Kaltumformung versteht man, dass das Werkstück vor der Umformung nicht über Raumtemperatur erwärmt wird, unter einer Warmumformung versteht man, dass das Werkstück über seine Rekristallisationstemperatur erwärmt wird und unter einer Halbwarmumformung versteht man, dass das Werkstück vor der Umformung erwärmt wird aber nicht über die Rekristallisationstemperatur.

Als Pressverfahren wird eine Säulenpresse mit einem Oberstempel und einer Matrize/Pressform vorgeschlagen.

Wie beschrieben, sollte die Partikelgröße des zu verpressenden Salzes vor der Fertigung ausgewählt werden und, wie erwähnt, im Bereich von 0,5 mm bis 2,5 mm liegen, idealerweise im Bereich 1 bis 1,6 mm (Durchmesser).

Die Auswahl der richtigen Partikelgröße ist deshalb wichtig, um eine optimale Befüllung der Pressform und Stabilität des Formkörpers 1 zu realisieren.

Die Schüttdichte der Salzpartikel in der Pressform/Matrize ist dabei von der Partikelgröße abhängig und in dem genannten Bereich die Partikelgröße noch ausreichend hoch. Bei größeren Partikeln kann es zu einer inhomogenen Füllung der Form und zu geringeren Schüttdichten und Instabilität des Formkörpers kommen, insbesondere kann es auch beim Verpressen dazu kommen, dass Lunker entstehen, also nicht mit Salz ausgefüllte Bereiche (Hohlstellen).

Die Masse (Gesamtgewicht) des erfindungsgemäßen Salzsticks 1 sollte im Bereich von 15 bis 40g liegen, ideal im Bereich von 25 bis 35g, besonders bevorzugt etwa 30g.

Dabei sollte die Länge L (x) des erfindungsgemäßen Formkörpers im Bereich von 50 bis 150 mm liegen, die maximale Breite im Bereich von 15 bis 30 mm und die maximale Höhe (Tiefe) ebenfalls im Bereich von etwa 15 bis 30 mm.

Eine Länge von etwa 100 mm und eine Breite und Höhe von jeweils 15 mm ist ideal.

Abweichungen von den vorgenannten Maßen können zu instabilen Formkörpern führen und Probleme bei der Produktion des Formkörpers ergeben, da der Formköper dann u.U. nicht mehr problemlos und unzerstört aus der Pressform entfernt werden kann.

Das ideale Oberflächen/Volumen Verhältnis des Formkörpers 1 sollte größer als 0,15 betragen (Zylinder 100 x 15) bevorzugt größer als 0,17 liegen (Zylinder 50 x 15).

Der erfindungsgemäße gepresste Formkörper 1 ist dabei so ausgelegt, dass er idealerweise bei einer typischen Dusch-, Handwasch- bzw. Badewaschtemperatur von z.B. etwa 34 bis 45°C innerhalb einer vorbestimmten Zeit, zum Beispiel 2 bis 10 Minuten, aufgelöst werden kann, wobei davon ausgegangen wird, dass die Durchflussmenge ca. 7,5 bis 20 l/min beträgt. Für weitere Anwendungen ist es auch möglich u.U. sinnvoll, die resultierende Salzkonzentration im Wasser noch weiter zu verringern, indem die Durchflussmenge verringert wird, sodass eine noch längere Auflösungszeit und damit Duschdauer erreicht wird. Dies ist u.U. vor allem bei einer Wellnessanwendung gewünscht.

Daraus ergibt sich dann eine resultierende Salzkonzentration im mit Salz angereicherten Wasser von 0,2 bis ca. 4 Gew%, optimal sind etwa 2 Gew%.

Dazu sei angemerkt, dass beispielsweise die Salzkonzentration des Wassers in der Nordsee bei etwa 3,5 % liegt, die Salzkonzentration von Wasser in der Ostsee im Bereich von 0,3 bis 1,8 % schwankt.

Bei Einsatz des gepressten Formkörpers in einer Kaltdusch, z.B. nach einem Saunagang zur Vitalisierung der Haut des Benutzers ist es auch möglich, dass der Formkörper so ausgelegt ist, dass er durch Wasser, welches nur eine Temperatur zwischen 1° und 15°C aufweist, aufgelöst wird. Je nach Duschzeit ist es dann dabei möglich, dass der Formkörper für eine solche Kaltdusche geringere Abmaße aufweist als ein Formkörper für die erwähnte typische Duschtemperatur von z.B. 34° bis 45°C.

Die Verkleinerung eines vorgefertigten Formkörpers zum Einsatz bei einer Kaltdusche ist einfach möglich, z.B. durch Verkürzung der Länge des Formkörpers auf das gewünschte Längenmaß, z.B. durch Zerteilung des erwähnten Formkörpers von 100mm Länge auf 3cm Länge.

Die Zerteilung kann durch ein Werkzeug, z.B. ein Messer durchgeführt werden aber auch durch ein Brechen des Formkörpers. Dieser kann außenseitig mit Markierungen versehen werden (die Markierungen sind den Figuren nicht dargestellt) um somit den Benutzer zu erleichtern, wo er den Formkörper zerbrechen muss, damit dieser für eine Kaltdusche in einer vorbestimmten Zeit aufgelöst werden kann.

Figur 4 zeigt eine weitere Darstellung des erfindungsgemäßen Applikators 2 in einer vergrößerten Ansicht. Der Applikator 2 weist einen Zulauf 8 und einen Ablauf 9 auf, die jeweils typische Normmaße aufweisen, z.B. ½ Zoll. Dabei weist der Applikator für den Zulauf 8 eine Schraubmuffe mit entsprechender Zollmaßigkeit auf, die sich auf einen entsprechenden Schraubanschluss der Standartarmatur aufschrauben lässt.

Wie in Figur 4 ebenfalls zu erkennen, weist der Abfluss 9 ein entsprechendes zollmaßiges Gewinde auf, auf den sich dann der Schlauch 4 mit einer entsprechend passenden Schraubmuffe aufschrauben lässt.

Darüber hinaus weist der Applikator 2 das Mehrwegeventil 7 auf, um den Durchfluss des Wassers durch den Applikator zu steuern. Bevorzugt ist das Mehrwegeventil ein Dreiwegeventil, erlaubt mithin also bis zu drei verschiedene Stellungen, sodass das Wasser, wie bereits erwähnt, den Flusspfad A, B oder A und B durch den Applikator nehmen kann.

In Figur 4 ist auch die Aufnahmekammer 5 zur Aufnahme des Formkörpers 1 gut zu erkennen. Diese Aufnahmekammer 5 weist wiederum eine Einlassseite 10 und eine Auslassseite 11 auf, sodass in die Aufnahmekammer 5 eindringende Wasser den festen Formkörper 1 umfließt bzw. umspült, dabei Salzpartikel des Formkörpers auflöst und das so mit Salz aufkonzentrierte Wasser verlässt auf der Abgabeseite 11 durch den an der Abgabeseite angeschlossenen Rücklauf bzw. Bypass 6 der Wiederaufnahmekammer und wird vom Bypass bzw. Rücklauf im Bereich des Auslasses 9 des Applikators 2 eingeleitet.

Die Aufnahmekammer 5 ist an ihrem freien Ende, dort wo der Auslass 11 angeordnet ist, mit einer Schraube bzw. Schraubmuffe verschließbar. Wird diese Schraubmuffe geöffnet, kann ein Formkörper in die Aufnahmekammer gebracht werden und durch Aufschrauben der Schraubmuffe 12 ein entsprechendes Außengewinde der Aufnahmekammer ist dann an dieser Stelle die Aufnahmekammer verschlossen, sodass das in die Aufnahmekammer 5 eindringende Wasser den Weg über den Einlass 10, Auslass 11 und Bypass 6 nehmen muss.

Für ein alternatives Aufschrauben des Verschlusses (der Muffe) auf der Aufnahmekammer sind andere Verbindungsarten und Verschlusstechnischen ebenfalls geeignet, z.B. Excenterverschluss, Schiebekeilverschluss, Schraubverschluss, Bajonettverschluss, Nutfederverschluss, Stopfen, etc. Entscheidend ist, dass in einer möglichst einfachen und möglichst einhändigen Bedienung die Aufnahmekammer nach Einsatz des Formkörpers dieser verschlossen werden kann.

Die Aufnahmekammer5 selbst besteht dabei aus einem transparenten Rohr 13. Das vorgenannte transparente Rohr kann beispielsweise aus Acrylglas, üblichem Glas, Polyvinylchlorid (PVC), beschichtetes PVC sein. Auch ist es möglich, statt einer Ausbildung eines transparenten Rohres ein blickdichtes Rohr mit einem entsprechenden Material auszubilden. Als blickdichtes Rohr ist dann auch ein Metallrohr, ein Kunststoffrohr, etc. möglich.

Der Bypass 6 weist bevorzugt ein Rückschlagventil, einen Rückflussminderer oder dergleichen auf, also ein Bauteil, um zu verhindern, dass bei einem normalen Duschvorgang Wasser über den Ausgang des Bypass 6 in die Aufnahmekammer 5 eindringt.

Durch die Bedienung des Mehrwegeventils 7 kann der Benutzer einstellen, ob das Wasser direkt durch den Applikator 2 fließt, ohne dabei die Aufnahmekammer 5 zu durchfließen (normaler Duschmodus) oder ob Wasser zunächst beim Eindringen in den Applikator 2 in die Aufnahmekammer 5 eindringt, dort den gepressten Formkörper umspült und diesen auflöst, um dann wieder zurück in den Applikator und in Richtung Duschkopf weiter zu fließen. Es ist auch möglich, dass sowohl Wasser direkt durch den Applikator einerseits als auch durch die Aufnahmekammer andererseits fließt (Mischmodus).

Das Mehrwegeventil kann als Zweiwegeventil aber auch alternativ als Dreiwege-, Vierwege- oder Mehrwegeventil ausgeführt werden. Das Ventil kann auch als elektromagnetisches oder mechanisches Ventil ausgeführt werden.

Der in den Figuren 3 bis 6 gezeigte Applikator 2 weist die Aufnahmekammer 5 auf, zur Aufnahme des gepressten Formkörpers (Salzstick). Diese Aufnahmekammer 5 weist innenseitig im Wesentlichen einen kreiszylindrischen Querschnitt auf und hat einen Durchmesser, welcher um ein lichtes Maß oder exakt so groß ist wie der größte Durchmesser (im Querschnitt y-z) des Formkörpers 1, wobei die Aufnahmekammer eine Länge L (x) aufweist, die etwas größer ist als die vorbestimmte Länge des gepressten Formkörpers 1, um somit ein gewisses axiales Spiel für den Formkörper 1 in der Aufnahmekammer 5 vorzusehen. Dieses axiale Spiel sollte größer sein als 1 mm, aber bevorzugt kleiner sein als 30 mm, besonders bevorzugt 15 mm. 5 mm erscheint ein guter Kompromiss zu sein.

Die Aufnahmekammer 5 selbst ist im dargestellten Beispiel ein Acrylrohr aus zum Beispiel transparentem Acrylglas mit einem Außendurchmesser von zum Beispiel 20 mm und einem geringeren Innendurchmesser, zum Beispiel 16 mm, wobei Abweichungen hiervor selbstverständlich ohne weiteres möglich sind. In den dargestellten Beispielen sind die in der Aufnahmekammer 5 untergebrachten gepressten Formkörper 1 so gestaltet, dass diese einen größten Querschnitt aufweisen, der im Bereich von 15,5 bis 15,8 mm liegt, woraus ersichtlich ist, dass sich diese gepressten Formkörper leicht in die Aufnahmekammer, also leicht in das Acrylrohr hineinschieben/einbringen lassen.

Wie erwähnt, sind die dargestellten Maße nur beispielhaft zu verstehen. Es ist auch ohne weiteres möglich, dass der Innendurchmesser der Aufnahmekammer bei 34 mm liegt, der Außendurchmesser dann entsprechend größer ist und der (maximale) Durchmesser des Sticks dann bei ca. 30 bis 33 mm liegt.

Die erwähnte Muffe/Schraube kann auch als Schieber, Stopfen, Hahn, etc. ausgebildet sein. Wenn in der Aufnahmekammer 5 in Sieb (nicht dargestellt) eingesetzt wird oder dieses Sieb auch am Innenteil der Muffe/Schraube 12, also des Verschlussstücks, angebracht ist, wird der Stick 1 gegen das Sieb gedrückt bzw. dieses Sieb bildet dann auch den Abstandshalter für das Sieb in Bezug auf den Auslass der Aufnahmekammer 5.

In den dargestellten Beispielen des Sticks 1 berührt dieser mit maximal vier Kanten die Innenseite der Aufnahmekammer 5, in einer anderen Ausformung des Sticks ist es auch möglich, dass der Sticks immer mit drei oder fünf oder sechs oder gar mehr Außenkanten die Innenseite der Aufnahmekammer 5, also des Acrylrohrs 13 berühren kann.

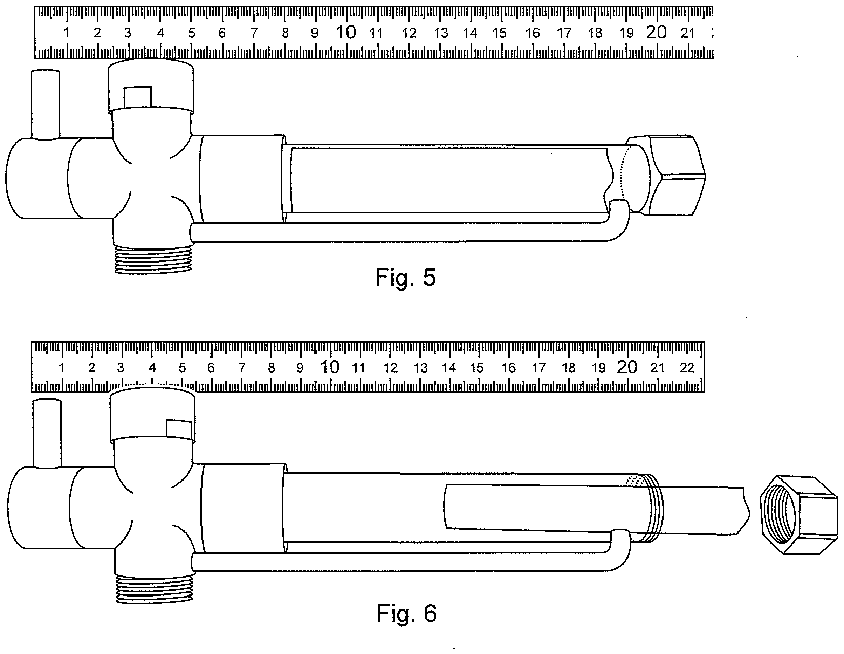

Wie in den Figuren 3 und 6 auch zu sehen, fließt das Wasser, nachdem es am Salzstick vorbeigeströmt ist, durch einen Rücklauf, einen sogenannten Bypass, zurück in die Armatur des Applikators.

Der Innendurchmesser DiB dieses Bypasses 6 ist bevorzugt geringer als der Durchmesser DiA der Aufnahmekammer 5, zum Beispiel nur halb so groß wie der Innendurchmesser der Aufnahmekammer 5, eine bevorzugte Größe für den Innendurchmesser des Bypasses 6 liegt im Bereich von 6 bis 10 mm, auch ein Durchmesser von 6 bis 20 mm liegt, ist möglich.

Schließlich ist auch vorgesehen, dass am Eingang des Bypasses, also am Auslassende 11 in der Aufnahmekammer 5, auch ein Sieb (nicht dargestellt) positioniert wird. Dies kann ein Siebeinsatz sein, der wie ein Formkörper in die Aufnahmekammer geschoben wird. Dieser Siebeinsatz hat den Vorteil, dass dann, wenn der gepresste Formkörper Bestandteile aufweist, die sich durch das Vorbeiströmen von Wasser nicht auflösen lassen, diese Bestandteile von dem Sieb zurückgehalten werden und diese Bestandteile nicht in den Bypass 6 und somit in den weiteren Leitungsverlauf für die Dusche gelangen kann.

Ein solches Sieb bietet sich insbesondere dann an, wenn Kräuter, Kräuterzusätze oder dergleichen in der Aufnahmekammer positioniert werden.

Dann ist es auch möglich, statt oder zusammen, als in Ergänzung mit dem gepressten Formkörper 1 auch unter Umständen Träger mit einem ätherischen Öl oder dergleichen in der Aufnahmekammer 5 unterzubringen. Ein solcher Träger mit ätherischen Ölen kann ein synthetischer Träger sein, der mit ätherischem Öl versetzt ist, aber auch Kräuter/Pflanzen, insbesondere Blätter, Blüten oder Stängel von Kräutern bzw. Pflanzen, z.B. Rosmarin, Thymian, Minze oder andere Kräuter, die dafür bekannt sind, dass sie bei Durchströmen mit warmen Wasser auch entsprechende Gerüche und wasserlösliche Inhaltsstoffe, (u.a. auch eventuell enthaltene ätherische Öle) die in den Kräutern enthalten sind, an das vorbeiströmende Wasser abgeben.

Selbstverständlich ist es auch möglich, dass der in der Aufnahmekammer5 untergebrachte Formkörper mit gewünschten ätherischen Ölen versetzt ist, die sich dann beim Auflösen des Formkörpers ebenfalls im Wasser anreichern, um somit beim Duschen eine angenehme Geruchsatmosphäre zu schaffen bzw. dem Benutzer die Möglichkeit gibt, sich eine Aromatherapie beim Duschen zu unterziehen.

Das Sieb hat dann dabei die Funktion wie ein "Teesieb", es verhindert also, dass unlösbare Kräuterbestandteile in den Bypass und in die weitere Leitung gelangen können (um dort eine Verstopfung auszulösen).

Bei 20°C können mit 100 ml Wasser ca. 35,8 g NaCl gelöst werden. Das bedeutet, dass man zur kompletten Auflösung eines Sticks relativ wenig Wasser benötigt, wenn das Wasser einen längeren Zeitraum die Möglichkeit hat, das Salz in seiner Kristallform aufzulösen.

Die Aufnahmekammer 5 weist regelmäßig ein Innenvolumen auf, welches geringer ist als das Volumen, welches mindestens notwendig ist, um den gepressten Formkörper 1 aufzulösen.

In dem dargestellten Beispiel nach den Figuren 4 bis 6 beträgt das Innenvolumen der Aufnahmekammer 5 ca. 80 ml.

Wenn der Stick 1 in der Aufnahmekammer 5 liegt, verringert sich entsprechend das freie Volumen für das Wasser.

Es ist auch möglich, das Volumen der Aufnahmekammer 5 so groß zu gestalten, dass nur mit einer einmaligen Befüllung derAufnahmekammer derdarin enthaltene gepresste Formkörper aus Salz sich auflöst. Ist dies geschehen, kann dann mit einmaligem Durchspülen der Aufnahmekammer die dort befindliche, relativ hoch konzentrierte Salz-Wasser-Lösung aus der Aufnahmekammer 5 herausdrückt werden, um somit zum Duschkopf zu gelangen.

Der Vorteil der Ausbildung der Aufnahmekammer 5 als transparentes Rohr 13 (z.B. aus Acrylglas, normales Glas (Fensterglas), PVC oder dergleichen) hat auch den Vorteil, dass damit die Aufnahmekammer für den Benutzer eine "Fensterfunktion" aufweist. Der Benutzer kann dann nämlich erkennen, ob der Salzstick 1 in der Aufnahmekammer sich schon aufgelöst hat oder nicht und wenn das Salz einen natürlichen Farbstoff aufweist, kann der Benutzer auch sehr gut erkennen, ob die Aufnahmekammer bereits "sauber gespült worden ist" oder ob dort noch Reste der Salz-Wasser-Lösung enthalten sind.

Da Alpensalz typischerweise eine braune Farbe als natürliche Färbung aufweist und auch andere Salze über eine natürliche Farbgebung verfügen, hat diese Sichtfensterfunktion der Aufnahmekammer auch die Bedeutung, dass der Benutzer selbst dafür sorgen kann, dass sehr sicher die gesamten Salzreste aus der Aufnahmekammer herausgespült worden sind.

Falls das Salz selbst nicht über eine natürliche Farbgebung verfügt, ist es selbstverständlich möglich, dass dem gepressten Formkörper im Pressvorgang auch ein Färbemittel beigefügt wird, welches sich dann bei Umströmung mit dem Wasser entsprechend auflöst.

Figuren 5 und 6 zeigen noch einmal weitere Ansichten des erfindungsgemäßen Applikators mit einem darin untergebrachten gepressten Formkörper 1 (Figur 5) sowie auch die Art und Weise, wie der gepresste Formkörper 1 in die Aufnahmekammer 5 des Applikators 2 eingebracht wird und danach mit der Schraubmuffe 12 die Aufnahmekammer 5 verschlossen wird.

Damit diese Schraubmuffe 12 nicht versehentlich nach dem Öffnen oder beim Verschrauben herunterfällt, kann diese mit einem entsprechenden Sicherungsring versehen werden, der einerseits das Öffnen und Verschließen als auch das Verdrehen der Schraubmuffe möglich macht aber über diesen Sicherungsring ist dann die Schraubmuffe verlierbar mit der Aufnahmekammer 5 oder dem Bypass 6 verbunden.

Wie ferner in Figur 3 gut zu erkennen ist, ist die Ausrichtung der Aufnahmekammer im installierten Bad in etwa in horizontaler Richtung, d.h. der Benutzer kann den gepressten Formkörper 1 im Wesentlichen horizontal in die entsprechend ausgerichtete Aufnahmekammer 5 des Applikators 2 schieben.

Dies verhindert ein unabsichtliches Herausrutschen des Formkörpers aus der Aufnahmekammer 5, falls nicht sofort nach dem Hineinschieben des Formkörpers 1 die Aufnahmekammer 5 diese mit der Schraubmuffe 12 verschlossen wird.

Ein weiterer Vorteil der im Wesentlichen horizontal ausgerichteten Anordnung der Aufnahmekammer besteht auch darin, dass dann der Benutzer beim Duschen selbst auch den Befüllungsgrad innerhalb der transparenten Aufnahmekammer 5 gut erkennen kann und es für ihn dann auch ein sehr Leichtes ist, das entsprechende Ventil 7 zu bedienen, weil er damit sofort merkt, über welche Stellung das Mehrwegeventil verfügt. Gerade diese einfache und im besten Sinne transparente Bedienbarkeit erhöht die Attraktivität des Systems.

Wenn der Benutzer den Applikator nicht mit einem Salzstick in der Aufnahmekammer befüllen möchte, sondern nur eine "Kräuterdusche" nehmen möchte, füllt der Benutzer vorab das erwähnte Sieb mit den gewünschten Kräutern, Kräuterbestandteilen, Kräuterzusätzen, ätherischen Ölen, etc. und führt dieses, wie den gepressten Formkörper, in die Aufnahmekammer 5 ein und verschließt die Aufnahmekammer 5 dann wiederum mit der Schraubmuffe 12.

Durch Einstellung der Temperatur des Wassers einerseits, wie auch der Wassermenge, kann dann auch der Benutzer einstellen, in welcher Konzentration die in den Kräutern enthaltenen ätherischen Öle bzw. Zusätze freigegeben werden, um ein angenehmes Geruchsklima in der Dusche zu bewerkstelligen.

Die vorliegende Erfindung ist, wie dargestellt, in vielfachen Anwendungen und Verwendungen möglich, z.B. in einer Dusche, in einem Bad oder dergleichen. Hierzu werden auch Wellnessanwendungen gezählt oder eine Anwendung, bei welcher der Benutzer sein Wohlbefinden steigert, weil z.B. durch Überstreichen von mit Salz oder anderen ätherischen Ölen oder Partikeln von Pflanzen, etc. angereicherten Wasser über der Haut das Wohlbefinden als solches steigert oder auch der Geruch bei der Anwendung vom Benutzer für sehr angenehm empfunden wird.

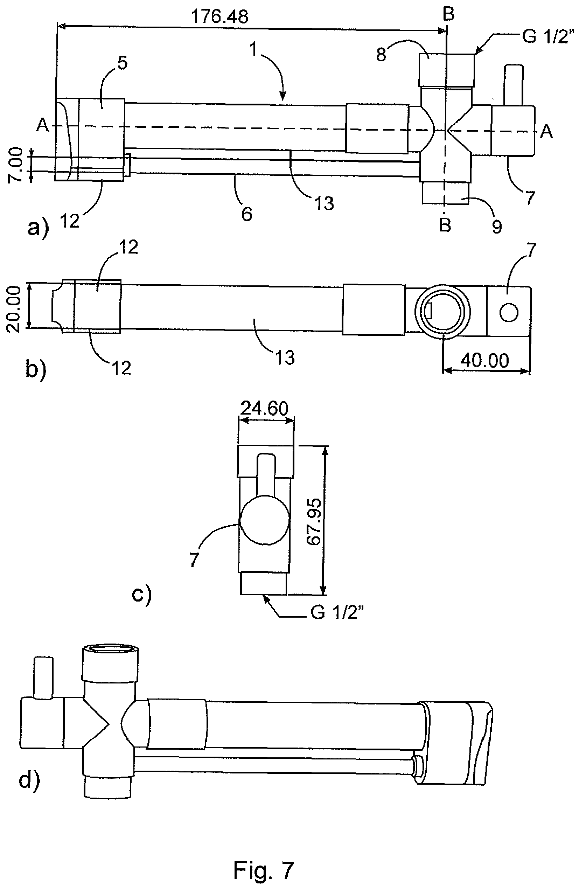

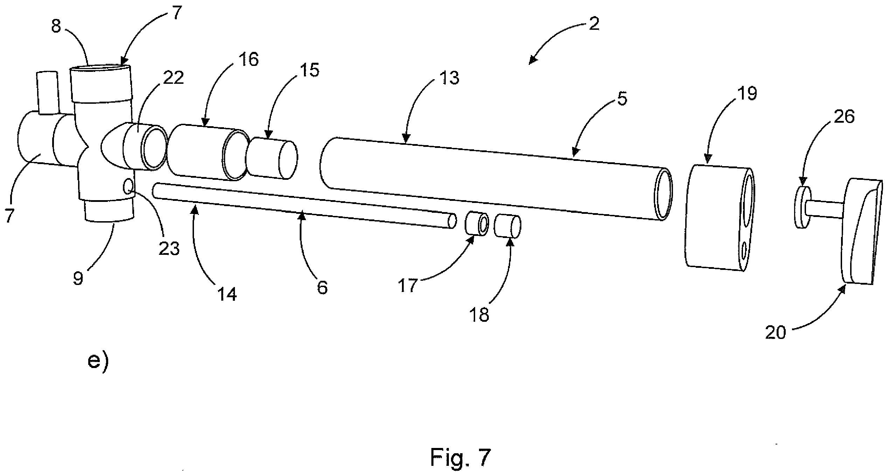

Figur 7 zeigt in 5 Darstellungen, Figur 7a, Figur 7b, Figur 7c, Figur 7d und Figur 7e eine weitere Darstellung eines weiteren Ausführungsbeispiels der Erfindung in verschiedenen Ansichten.

Der Grundaufbau der Ausführungsform nach Figur 7 ist dabei der gleiche wie nach den Figuren 3 bis 6.

Figuren 7a, b, c, und d zeigen den erfindungsgemäßen Applikator aus verschiedenen Ansichten und mit weiteren Details der Ausführungsform. Weitere Details sind insbesondere in Figur 7e in der Explosionsdarstellung besser erkennbar.

Wie bereits beschrieben, weist der Applikator ein Dreiwegeventil 7 auf mittels welchem drei verschiedenen Einstellungen grundsätzlich möglich sind (wobei auch auf die eine oder andere Einstellung verzichtet werden kann), nämlich der direkte Durchfluss von Wasser am Eingang 8 des Applikators zum Ausgang des Applikators 9 oder der Durchfluss von Wasser über den Eingang 8 in die Aufnahmekammer 5 und dann zurück über den Bypass 6 zum Auslass 9 oder auch eine Mischfunktion, wobei dann das Wasser sowohl direkt vom Einlass 8 zum Auslass 9 fließt, als auch vom Einlass 8 über die Aufnahmekammer 5 über die Aufnahmekammer 5 über den Bypass 6 zum Auslass 9.

Zur Befestigung der Aufnahmekammer 5, die im dargestellten Beispiel als Acrylglas ausgeführt ist, dient eine Gewindemuffe 16 mit Ansatzfräsung und zum Einkleben des Acrylglasrohres in dieser Gewindemuffe einerseits und zum Anschrauben an ein entsprechendes Gegengewinde am Abgang 22. Ferner ist in der Muffe 16 ein Rückflussverhinderer 15 ausgebildet, der verhindert, dass Wasser vom Auslassbereich 9 über den Bypass 6 und die Aufnahmekammer 5 zurück in den Ventilteil 7 gelangen kann.

Das Acrylglasrohr 13 hat hohl zylindrische Abmaße und wird unterseits mit einem Deckel 19 verschlossen, der auch einen Anschluss für den Bypass 6 aufweist. In den Deckel ist von außen ein Sieb und Verschluss 20 einsteckbar, sodass Wasser, welches für den Abgang 22 des Ventils in die Aufnahmekammer 5 gelangt, durch das Sieb treten muss, bevor es über den Bypass 6 zum Ausgang 9 zurückfließt.

Zur mechanischen Aufnahme und Abdichtung des Bypass 6 sind eine Einklebemuffe 17 und ein Rückflussverhinderer 18 vorgesehen, sodass Wasser vom Bereich des Abgangs 9 nicht über den Bypass 6 in die Aufnahmekammer 5 fließen kann.

Im dargestellten Beispiel ist der Bypass 6 als Edelstahlrohr 14 ausgebildet, welches bevorzugt auf verchromt sein kann. Dieses Edelstahlrohr 14 ist in eine entsprechende Bohrung 23 des Ventilteils 7 eingelassen und dort dichtend befestigt.

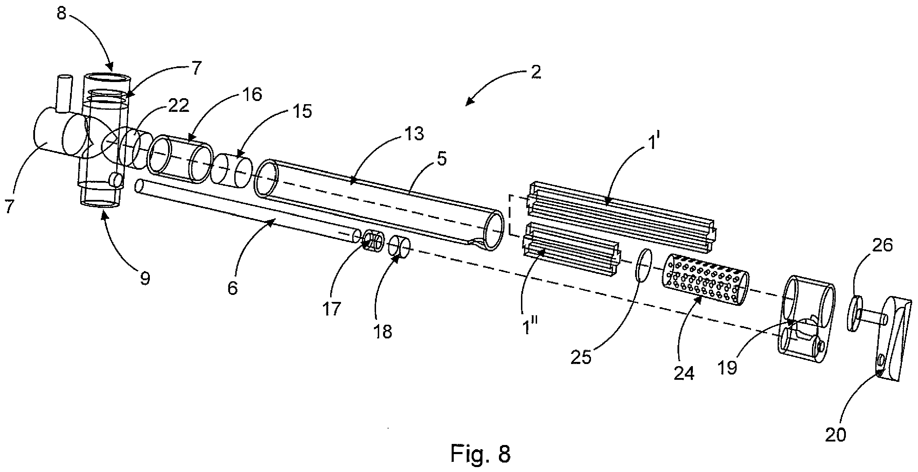

Figur 8 zeigt die bereits in Figur 7 beschriebene Anordnung, jedoch mit weiteren Details. In Figur 8 ist auch der Formkörper 1 dargestellt, der in die Aufnahmekammer 5 eingeschoben werden kann. Im dargestellten Beispiel sind zwei verschiedene Formkörper 1' bzw. 1" dargestellt. Während der erste Formkörper 1' eine Normallänge für einen normalen (z.B. 3 - 5 Minuten bei 38°C) Duschgang aufweist, ist der zweite Formkörper 1" verkürzt, beispielsweise für einen Kurzduschgang, z.B. auch für eine Kaltdusche nach einem Saunagang (z.B. ca. 90 Sekunden - 180 Sekunden bei weniger als 20°C, vorzugsweise weniger als 15°C).

In der Darstellung in Figur 8 ist auch ein Siebkörper dargestellt, der in die Aufnahmekammer 5 geschoben werden kann. Dieser Siebkörper 24 weist einen oberseitigen Deckel 25 auf, sowie einen unterseitigen Siebdeckel 26, der Teil des Verschlusses 20 der Aufnahmekammer ist.

Das Sieb ist dazu geeignet, einerseits nicht auflösbare Bestandteile, die sich u.U. im Formkörper befinden, zurückzuhalten. Ferner ist das Sieb 24 dazu geeignet, Pflanzenteile, z.B. von Kräutern, etc. oder auch andere auflösbare Bestandteile (z.B. Öle, etc.) aufzunehmen. Das Sieb 24 kann zusammen mit dem Formkörper 1', insbesondere 1" in der Aufnahmekammer 5 untergebracht werden, aber auch allein. Durch die ober- und unterseitigen Deckel 25 und 26 dies Siebkörpers 24 werden insbesondere nichtlösliche Bestandteile, die innerhalb des Siebs untergebracht werden und die bei einem Duschgang vom Wasser umspült werden, im Sieb zurückgehalten und können somit nicht in den weiteren Wasserfluss über den Bypass 6 etc. in das Duschwasser gelangen und somit keine Verstopfung einer Leitung verursachen.

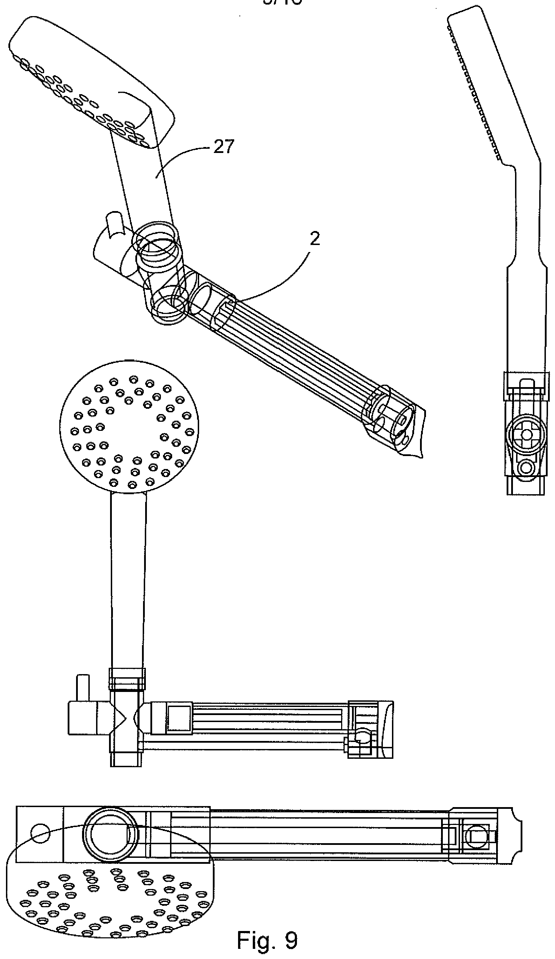

Figur 9 zeigt eine weitere Variante der Erfindung, bei der ein Duschkopf 27 direkt am Ausgangsanschluss 9 des Applikators 2 angebracht, z.B. verschraubt ist. Dabei kann der Applikator auch vom Benutzer als Handgriff für den Duschkopf verwendet werden, wenn beispielsweise der Benutzer den Teil der Aufnahmekammer umfasst. Im dargestellten Beispiel nach Figur 9 ist der Schlauch an der Eingangsseite 8 des Applikators 2 nicht dargestellt.

Die in Figur 9 dargestellte Kombination vom Duschkopf 27 einerseits und Applikator 2 andererseits ist ohne weiter Hilfsmittel miteinander zu verbinden, weil der Duschkopf regelmäßig ebenfalls über einen zollmaßigen (z.B. ½ Zoll) Normanschluss verfügt wie der erfindungsgemäße Applikator 2.

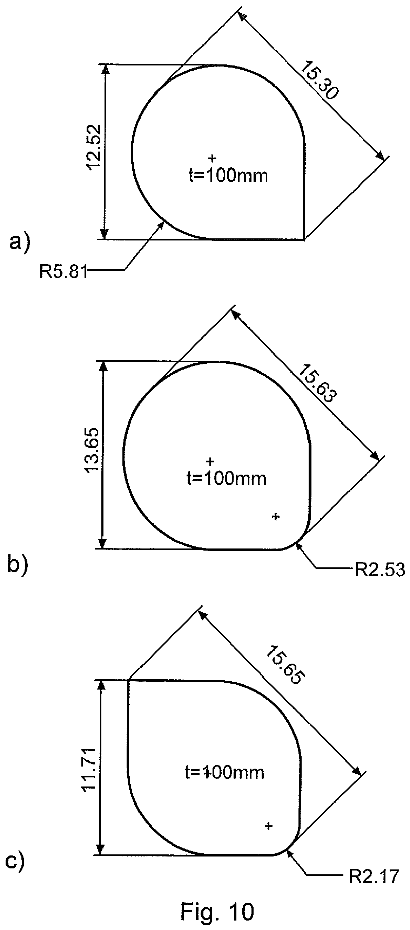

Figur 10 zeigt einen Querschnitt von drei weiteren Varianten des Formkörpers, wobei die dargestellten Querschnitte nicht rotationssymmetrisch sind. Die Beispiele gemäß der Figuren 10 a), 10b) und 10c) sind spiegelsymmetrisch. Auch eine völlige unsymmetrische Form wäre ohne weiteres denkbar.

Die in den Zeichnungen dargestellten Maße sind, wie in allen Zeichnungen, beispielhaft zu verstehen und dienen lediglich dem besseren Verständnis des Fachmanns.

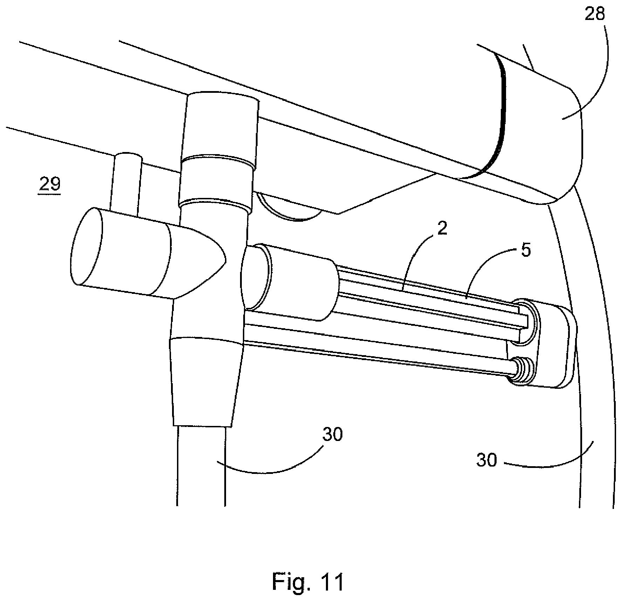

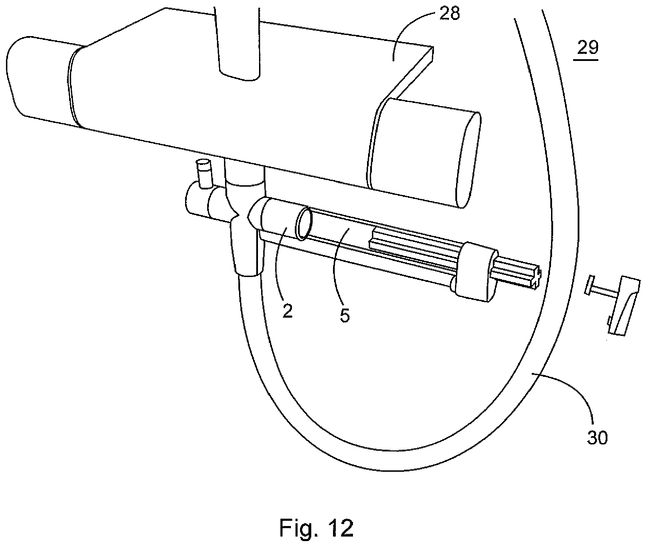

Die Figuren 11 und Figur 12 zeigen nochmals den Applikator 2 im bevorzugtem Einbau an einer bestehenden Duscharmatur 28, die an der Wand 29 befestigt ist. Ausgangsseitig des Applikators 2 ist ein Schlauch 30 angeschlossen, an dessen freien Ende sich der eigentliche Duschkopf befindet.

Wie in den Figuren 11 und 12 zu erkennen, ist der Applikator 2 so unterhalb der Armatur 28 angeordnet, dass die Aufnahmekammer in ihrer Längsrichtung im Wesentlichen horizontal ausgerichtet ist, sodass der Benutzer stets sehr gut erkennen kann, ob die Aufnahmekammer 5 mit einem Formkörper 1 und/oder Sieb 2 bestückt ist und falls sich ein Formkörper 1 in der Aufnahmekammer 5 befindet, inwieweit dieser Formkörper durch den Wasserzufluss bereits aufgelöst ist.

Soweit der Formkörper ein farbiges Salz enthält, ist auch die Farbe des Salzes u.U. im Wasser erkennbar.

Statt wie in den Figuren 11 und 12 unterseits der Armatur, ist es auch ohne weiteres möglich, den Applikator 2 oberhalb der Armatur entsprechend anzuschließen.

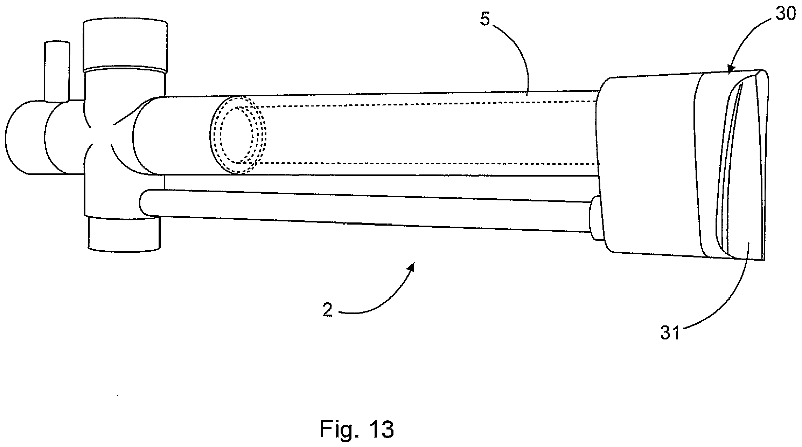

Figur 13 zeigt nochmals einen zusammengebauten Applikator 2 mit einer unbestückten Aufnahmekammer 5. In Figur 13 ist auch gut zu erkennen, dass der Verschluss 20 eine Griffmulde 31 aufweist, damit der Benutzer den Verschlussdeckel gut von der Deckelaufnahme bzw. Bypassanschluss 19 lösen bzw. befestigen kann.

Es ist erfindungsgemäß auch möglich im Applikator 2 einen oder mehrere Sensoren unterzubringen, beispielsweise einen Sensor zur Messung der Wassertemperatur, einen Sensor für Wasserfluss (l/min), einen Sensor zur Messung der Duschdauer oder auch zur Messung des im Wasser gelösten Salzgehaltes o.a. Sensoren. Die von dem Sensor oder den Sensoren erfassten Daten können in einem ebenfalls im Applikator 2 untergebrachten Datenspeicher zwischengespeichert werden und/oder über eine Kommunikationsschnittstelle z.B. Bluetooth, Ethernet, WLAN oder dergleichen an eine Datenverarbeitungseinheit, z.B. ein Smartphone, Tablet, Computer, etc. weitergeleitet werden, um dort einerseits gespeichert zu werden, andererseits verarbeitet zu werden und/oder entsprechende Auswertungen dem Benutzer anzuzeigen.

Für die Energieversorgung der Sensoren und/oder Datenkommunikationseinheit oder Speicher eignen sich Batterien, Akkumulatoren, bevorzugt aber thermoelektrische Generatoren (TEGs), die in der Lage sind, aus Wärme elektrische Energie (Strom) zu erzeugen, die dann ausreicht, um die Sensoren, Datenkommunikationsschnittstelle, Speicher zu betreiben. Da beim Duschgang regelmäßig ausreichend durch das vorhandene warme Wasser ein hinreichend großer Temperaturunterschied erzeugt wird, ist der Einsatz bzw. die Verwendung der thermoelektrischen Generatoren auch deshalb vorteilhaft, weil damit die gesamte Einheit aus der digitalen Schnittstelle, also Speicher, thermoelektrischer Generator, Datenkommunikationseinheit, Sensoren, etc. wasserdicht und wenn gewünscht auch unlösbar im Applikator untergebracht werden kann.

Thermoelektrische Generatoren, die für den Einsatzzweck geeignet sind, sind bekannt beispielsweise von der Homepage www.otego.de.

Als elektrischer Zwischenspeicher für die von dem thermoelektrischen Generator erzeugten elektrischen Energie eignet sich eine Akkumulatorzelle aber auch ein Superkondensator vom Ultracap-Typ. Soweit beim Wasserfluss ausreichend elektrische Energie in den Akkumulator gespeichert ist, wird dies durch eine Messschaltung festgestellt und sodann können durch die Sensoren die gewünschten Daten, Parameter, etc. (z.B. Temperatur, Duschdauer etc.) erfasst werden und über den eingerichteten Kommunikationskanal, z.B. Bluetooth, Ethernet, WLAN oder dergleichen, können die Daten an eine vorgesehene Datenverarbeitungseinrichtung, wie z.B. Smartphone, Tablet, Computer oder dergleichen versendet werden und/oder an eine Anzeigeeinrichtung des Applikators oder dergleichen angezeigt werden.

Erfindungsgemäß ist es auch möglich, dass, sobald die Sensoren einen Wasserfluss feststellen, diese Daten über den eingerichteten Kommunikationskanal an einen Datenempfänger, wie ein Smartphone, Tablet, Computer, PC, etc. geschickt werden und dort die weitere Auswertung stattfindet, wie lange der Wasserfluss stattfindet und mit welchem Temperaturprofil der Duschvorgang verlief, wie hoch die Salzkonzentration war, etc.

Falls der Benutzer eine Mindestduschzeit vorgeben möchte, kann dies bei Ablauf der Duschzeit durch ein entsprechendes optisches und/oder akustisches Signal dem Benutzer mitgeteilt werden. Das optische Signal könnte beispielsweise ein Signal auf dem Smartphone, Tablet oder Computer sein, welches in Sichtweite des Benutzers angeordnet ist oder eine Signalleuchte, die im/am Applikator oder Duschschlauch untergebracht/angebracht ist und ebenfalls durch den thermoelektrischen Generator oder einen entsprechenden Akku mit elektrischer Energie versorgt wird.

Wenn beispielsweise der Benutzer eine Duschzeit von 4 Minuten eingestellt hat, beginnt der Signalgeber nach 4 Minuten der Signalabgabe.

Dieser Signalgeber hat auch den Vorteil, dass dann, wenn die zeitliche Dauer bis zur Signalabgabe eingestellt werden kann, der Benutzer selbst sein Verbrauchs- bzw. Duschverhalten steuern kann, da der Benutzer dann, wenn die Signalabgabe erfolgt, z.B. rotes Blinken oder ein entsprechender Signalton, den Duschvorgang beenden wird. Somit trägt die Erfindung auch maßgeblich zur Wasser- und Energieersparnis bei.

Es sei angemerkt, dass die Ausstattung des Duschkopfes und/oder des Applikators und/oder des Duschschlauchs mit z.B. Sensoren, Datenspeicher, Anzeigeeinrichtung (z.B. LED-Anzeige) Energieversorgung, insbesondere mit einem thermoelektrischen Generator, Datenzwischenspeicher, etc. auch Ausführungsform der Erfindung ist, die unabhängig eigenständig die Ausbildung des erfindungsgemäßen Applikators bzw. des erfindungsgemäßen Formkörpers erfolgen kann, allerdings ist die Unterbringung der Sensoren und ihre hauptgeschilderte Datenkommunikation, insbesondere zur Anzeige der maximalen Duschdauer, der Wassertemperatur, etc. ein technische Ergänzung des erfindungsgemäßen Applikator bzw. Formkörper, um insoweit ein gewünschtes Wellness-Duschempfinden zu ermöglichen.

Dann, wenn der gepresste Formkörper durch das umspülende Wasser in der Aufnahmekammer 5 des Applikators 8 aufgelöst ist und dies dem Benutzer angezeigt wird, kann der Benutzer sofort den Duschvorgang beenden, weil einerseits sämtliches Salz in der Aufnahmekammer aufgelöst ist und andererseits sich noch Salz angereichertes Wasser auf seiner Haut befindet und noch nicht durch klares, ungesalzenes Duschwasser abgespült ist. Da beim Abtrocknen nicht sämtliches Wasser und nicht sämtliches im Wasser befindliches Salz vom Körper entfernt wird, kann somit auch ein gewisser Restsalzgehalt auf der Haut verbleiben, wenn dies vom Benutzer gewünscht wird. Ist dies vom Benutzer nicht gewünscht, hält er den Duschvorgang einfach länger an und sämtliches Salz-Wasser wird von der Haut gespült.

Falls der Benutzer nach dem Duschvorgang auf seiner Haut noch Salz angereichertes Wasser trägt und er dieses im Handtuch abtrocknet, wird auch entsprechend etwas Salz in das Handtuch gelangen und nach dem Trocknen des Handtuchs im Handtuch selbst wiederum verbleiben. Dies hat u.U. den Vorteil, dass insoweit das Handtuch nicht so schnell von Keimen und Mikroorganismen besiedelt wird, weil die Salzkristalle für Keime und Mikroorganismen lebensfeindlich sind und diese töten werden oder wenigstens ein weiteres Wachstum im Feuchteklima des Handtuchs verhindern.

Es ist erfindungsgemäß auch möglich, dass der oder die Sensoren, die Anzeigeeinrichtung, die Kommunikationsschnittstelle und die Energieversorgung in einer Einheit untergebracht sind und ein integraler Bestandteil des Duschkopfes, des Applikators 8 oder der Leitung zwischen Applikator und Duschkopf, z.B. dem Duschschlauch sind.

Dabei hat die Unterbringung der Einheit in einem Schlauch oder als Installationsmodul am Schlauch oder Applikator den Vorteil, dass der Benutzer die Nachrüstung eines Applikators mit der Datenkommunikation durch z.B. einen Austausch bzw. Installation eines entsprechenden Schlauchs oder Einheit vornehmen kann, in dem der oder die Einrichtungen wie z.B. Sensoren, die Kommunikationsschnittstelle, Anzeigeeinrichtung, Zwischenspeicher, Energieversorgung, etc. untergebracht sind.

Wenn in der Aufnahmekammer zusätzlich zum Formkörper Pflanzenteile, z.B. Blätter, Blüten oder Stängel von Pflanzen, insbesondere Kräutern, z.B. Rosmarin, Thymian, etc. untergebracht werden, insbesondere von Pflanzen, die bei Umspülung mit Wasser Inhaltsstoffe abgeben, hat dies für den Benutzer den Vorteil, dass diese Inhaltsstoffe beim Duschen über die Haut des Nutzers streichen /fließen und dort eine gewünschte hautpflegende Wirkung erzielen können. Oftmals sind diese Wirkstoffe auch dann, wenn sie sich unter Wärme verflüchtigen (odorisieren), auch für den Benutzer sehr angenehm, weil der Benutzer dann diese verflüchtigten, also im Duschwasser enthaltenen Wirkstoffe in einer Art "Duschwassernebel" inhalieren kann. Dies hat beispielsweise bei der Verwendung von Minzeblättern in der Aufnahmekammer des Applikators zur Folge, dass der Benutzer den Eindruck hat, die frischen ätherischen Öle der Minze zu riechen und zu atmen, was von vielen Benutzern als sehr angenehmer Geruch wahrgenommen wird und auch einen pflegenden und heilenden Effekt für Atemwege bzw. Nasen-, Mund- und Rachenraum darstellen kann.

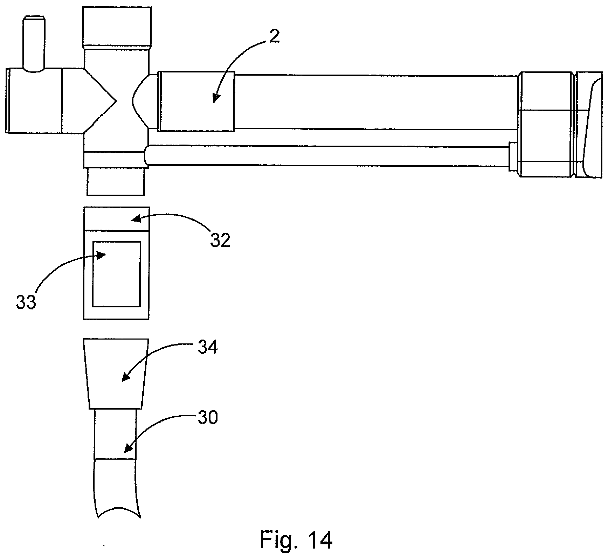

Figur 14 zeigt ein Beispiel eines erfindungsgemäßen Applikators bzw. einer erfindungsgemäßen Duscheinrichtung, bei welcher auf der wasserabgangsseitigen Seite am Ausgang 9 des Applikators 2 eine mit Rohröffnung versehene Einheit 32 angeschraubt ist. Diese Einheit 32 enthält bevorzugt einen thermoelektrischen Generator, also eine Einrichtung zur Erzeugung elektrischer Energie. In dieser Einheit 32 können auch ein oder mehrere Sensoren untergebracht werden, z.B. zur Messung der Wassertemperatur, des Salzgehalts des Duschwassers, etc., darüber hinaus kann dort auch der Beginn eines Duschvorgangs zeitlich gemessen werden und auch die Zeitdauer des Duschgangs ermittelt werden.

Diese Einheit 32 weist darüber hinaus eine Anzeigeeinrichtung 33 auf, z.B. in Form eines wassergeschützten LED-Elements, auf dem die gemessenen Werte der Sensoren angezeigt werden. Wenn diese Anzeigeeinheit eine Bildausgabequelle mit Touch-Funktion ist, kann durch wiederholtes Berühren der Anzeige eine Änderung der jeweiligen Anzeige bewirkt werden, z.B. durch wiederholtes Berühren können verschiedenste gemessene Parameter angezeigt werden, wenn die gleichzeitige Anzeige aller Parameter zu unübersichtlich erscheinen sollte. Falls die Anzeigeeinrichtung kommunikationstechnisch gekoppelt ist mit einer Steuereinrichtung, nämlich einer Steuereinrichtung zur Steuerung eines Ventils, welche in einem Schlauch oder einer Wasserleitung angeordnet ist, ist es auch möglich, durch eine entsprechende Eingabe an der Anzeigeeinrichtung das Ventil zu öffnen oder zu verschließen, um somit den Wasserfluss zu ermöglichen bzw. zu Stoppen oder mit einer entsprechenden Eingabe den gewünschten Wasserfluss in einer gewünschten Menge einzustellen. Dies hat den Vorteil, dass die Einstellung u.U. präziser erfolgt als mit den bisherigen Armaturen, mit denen nicht jeder Benutzer zugleich vertraut ist und wo es dann zu Fehleinstellungen kommt. Wenn mit der Anzeigeeinrichtung die Kommunikation mit einer Ventilsteuerung möglich ist, die auch gleichzeitig ein Mischventil darstellt, kann auch über die Anzeigeeinrichtung die gewünschte Temperatur eingestellt werden. Hier liegt der Vorteil besonders darin, dass tatsächlich eine Temperatur eingestellt werden, die sich nicht erst durch das Gefühl des Wassers auf der Haut einstellt, sondern vom Benutzer selbst gewünscht wird.

Wenn beispielsweise an einer bisherigen Armatur eine Temperaturvon 38° eingestellt wird, ist das die Temperatur, mittels der das Wasser die Armatur z.B. Zwischenteil verlässt. Wenn das Wasser durch den Duschschlauch und den Duschkopf fließt, fällt es vom Duschkopf herunter auf die Person, die sich duscht und auf dem Weg vom Mischventil bis zur Person gibt es regelmäßig einen erheblichen Abfall der Temperatur. Die Temperatur, mit der dann das Wasser die Haut erreicht, hängt auch maßgeblich davon ab, welche Temperatur im Duschbereich herrscht, wie groß die Person ist, denn der größte Temperaturabfall stellt sich für das Wasser vom Duschkopf bis zum Auftreffpunkt auf der Haut der Person ein, d.h. dass die Wassertemperatur dann für kleinere Personen häufig eher zu kühl ist als für größere Personen, deren Kopf sehr nahe am Duschkopf liegt.

Thermoelektrische Generatoren als solche sind schon bekannt und beruhen regelmäßig auf der gegenseitigen Beeinflussung von Temperatur und Elektrizität. Solche thermoelektrischen Generatoren beruhen auf dem Seebeck-Effekt (auch thermoelektrischer Effekt genannt), sind auch Peltier-Effekt oder Thomson-Effekt bekannt. Da bei Durchströmung mit warmen Wasser hinreichend Wärme an dem thermoelektrischen Generator vorbei fließt, reicht dies regelmäßig schon aus, um die notwendige elektrische Energie zum Betrieb der Sensoren aufzubringen. Ohne weiteres kann in der Einheit 32 auch noch ein Kondensator, ein Akkumulator oder dergleichen untergebracht werden, um somit elektrische Energie zu speichern, die nicht sogleich über die Anzeige bzw. für den Betrieb der Sensoren notwendig ist.

In der Einheit 32 kann auch eine bi- oder omni-direktionale Kommunikationseinheit, z.B. für Bluetooth oder WLAN-Kommunikation untergebracht werden, um die entsprechenden Daten der Sensoren an einen Empfänger, z.B. ein Smartphone, Tablet, Computer, etc. zu versenden.

Der Vorteil der in Figur 14 gezeigten Lösung besteht darin, dass die Einheit 32 ein separates Element ist, welches ausgangsseitig über eine Verschraubung mit dem Ausgang 9 des Applikators verbunden werden kann, auf der dann entsprechenden Ausgangsseite der Einheit 32 kann wiederum eine normierte Überwurfmutter 34 des Duschschlauches 30 angebracht werden.

Der Vorteil der gezeigten Lösung besteht daher insbesondere darin, dass der Kunde selbst entscheiden kann, ob er die Einheit 32 mit der eingebauten Sensorik und Anzeige verbaut haben möchte oder nicht.

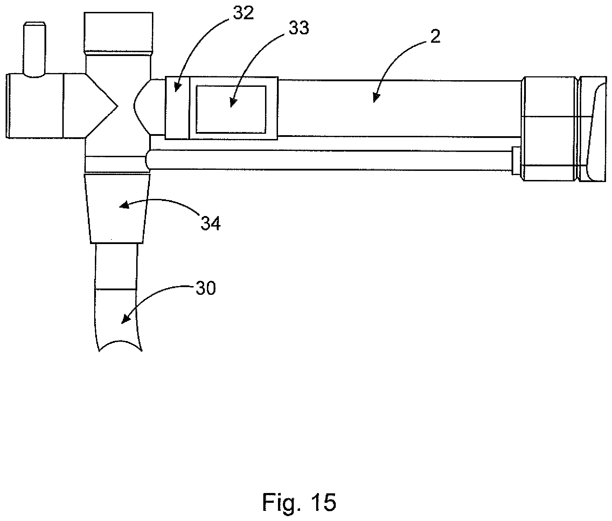

Figur 15 zeigt eine etwas andere Lösung als die in Figur 14 gezeigte. Bei Figur 15 ist nämlich die Einheit 32 mit einem thermoelektrischen Generator, der Bildausgabequelle, also dem Monitor (Anzeigeeinrichtung), der Sensorik, etc. integraler Bestandteil des Applikators 2. Dabei ist es auch möglich, dass die Einheit 32 dem Acrylrohr/Glasrohr 13 übergestülpt wird und auf diesem verschiebbar ist.

Die Einheit 32 mit dem thermoelektrischen Generator oder einer entsprechenden anderen Energieerzeugungseinrichtung oder einem entsprechenden Energiespeicher, z.B. Akkumulator oder Batterie, ist auch eine eigenständige Erfindung, die zwar hervorragend mit dem Applikator 2 zusammen passt aber auch völlig eigenständig ohne Applikator 2 ausgebildet eingesetzt werden kann. Erfindungsgemäß kann die Einheit 32 daher auch unabhängig von den anderen Erfindungen, die in der vorliegenden Anmeldung beschrieben sind, beansprucht werden. Die entscheidenden Merkmale der Einheit 32 bestehen darin, dass einerseits eine elektrische Energiequelle zur Verfügung steht um den Betrieb des in der Einheit 32 vorgesehenen mindestens einen Sensors, z.B. ein Sensor zur Messung der Wassertemperatur, des Salzgehalts des Duschwassers, etc. und die Einheit 32 z.B. auch über eine Anzeigeeinheit 33 verfügt, z.B. ein LED-Element und die elektrische Energiequelle auch die Anzeigeeinheit elektrisch versorgt, um gewünschte Anzeigen vorzunehmen. Ferner weist die Einheit 32 auch eine Schaltung auf, um die Sensordaten nicht nur zu messen, sondern auch auszuwerten und/oder für die Anzeige auf der Anzeigeeinheit aufzubereiten. Hierbei kann die Einheit 32 auch über eine Kommunikationsschnittstelle verfügen, z.B. Bluetooth, WLAN, etc., um die vom mit einem Sensor gemessenen Daten an einen weiteren Empfänger zu senden, z.B. ein Smartphone oder dergleichen.

Als Material für die Aufnahmekammer kommt, wie erwähnt, neben kristallinem Glas auch ein Acrylmaterial, z.B. Acrylglas in Frage aber auch ein Material auf Basis von Polyethylen (PE) oder PVC-U, also Hart-PVC (PVC - Polyvinylchlorid - Unplasticised) oder entsprechend anderes alternatives Kunststoffmaterial, welches insbesondere dafür geeignet ist, eine stabile Aufnahmekammer 5 zu bilden, die auch ein hohes Maß an optischer Transparenz aufweist.

In den Tabellen I bis IV sind Produktspezifikationen für verschiedene Salze aufgezeigt, die geeignet und bevorzugt sind, als Salzpartikel in den gepressten Formkörper Einsatz und Verwendung zu finden. Hervorragende Alternativen hierzu sind auch andere Salze, z.B. Salz aus dem Toten Meer (Totesmeersalz), Mittelmeersalz, Andensalz, u.ä.

Soweit in der vorliegenden Anmeldung der Einsatz und die Verwendung bestimmter ätherischer Öle verwendet wird, sind damit insbesondere ätherische Öle von Pflanzen wie z.B. Lavendel, Ylang Ylang, Eukalyptus oder dergleichen gemeint. Als Öl wird insbesondere "EUCALYPTUS RADIATA, Afrique du Sud, Lot N 406006 (Eucalyptus radiata leaf/stem oil)", "Lavandula angustifolia Miller (Lavandula officinalis Chaix, Lot 5360349", "Cananga odorata (YLANG COMPLETE Mada BIO FFL), Lot B975F007" als besonders bevorzugt und erfinderisch geeignet herausgestellt.

Der Anteil des Öls als Additiv im gepressten Formkörper sollte nach einer bevorzugten Ausführungsform der Erfindung der vorliegenden Anmeldung im Bereich von 0,5-4 Gew.% liegen, optimal sind 1-2 Gew.%.

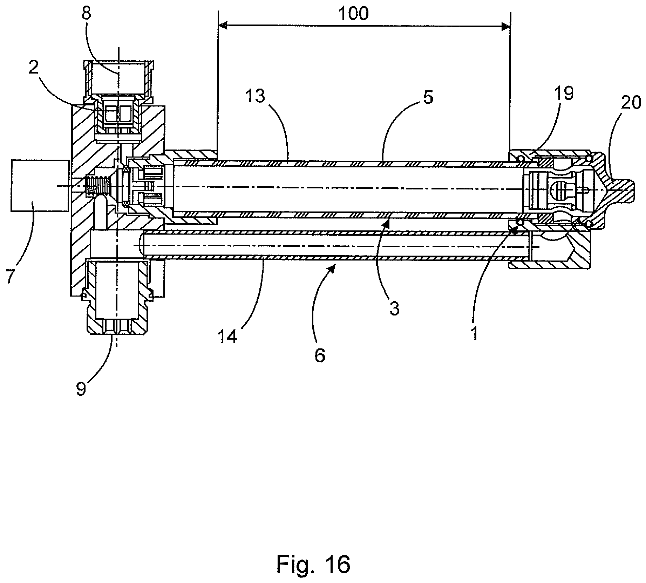

Figur 16 zeigt eine weitere Möglichkeit des Aufbaus des Applikators, wobei der Aufbau dem Fachmann ohne weiteres verständlich ist.

Nachstehend werden Produktspezifikationen für verschiedene Salze als auch Additive aufgeführt, die jeweils besonders gut geeignet sind, erfolgreich einen stabilen Salzstick herzustellen und bei Bedarf mit den gewünschten Additiven zu versetzten, wobei das jeweils eingesetzte Additiv auch die Funktion aufweisen kann, dass die einzelnen Salzpartikel noch besser zusammenhaften. Das Additiv hat also auch eine Funktion, den Salzstick insgesamt mechanisch zu stabilisieren und andererseits dann, wenn der Salzstick von Wasser umflossen wird, nicht nur das Salz aufgelöst, sondern auch das Additiv freigesetzt, was in der Dusche einerseits einen angenehmen Geruch produziert, der beim Einatmen auch gesundheitsfördernd sein kann und andererseits werden auch die im Wasser gelösten Bestandteile des Additivs die Hautberührung vom Benutzer als sehr angenehm und gesundheitsförderlich empfunden.

Als Additiv ist es erfindungsgemäß auch möglich ein Tensid oder Lipid oder eine Fettphase aus z. B. Tensid und / oder Lipid dem Salzstick beizumengen, um somit den Salzstick auch als Duschgelstick einsetzen zu können mit einer Duschreinigungsfunktion für Haut oder Haare.

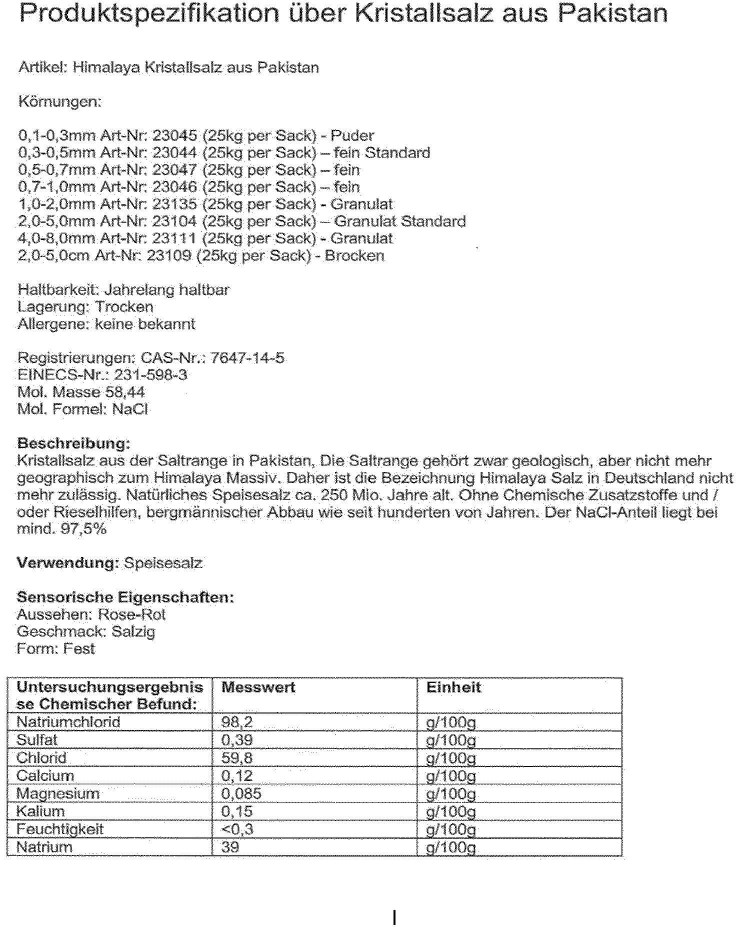

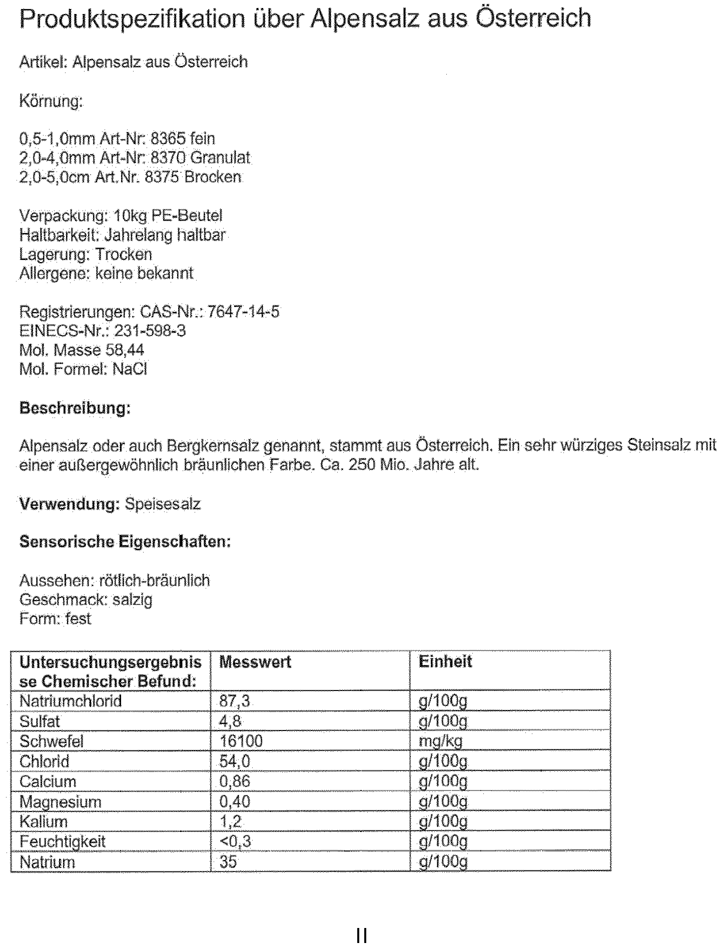

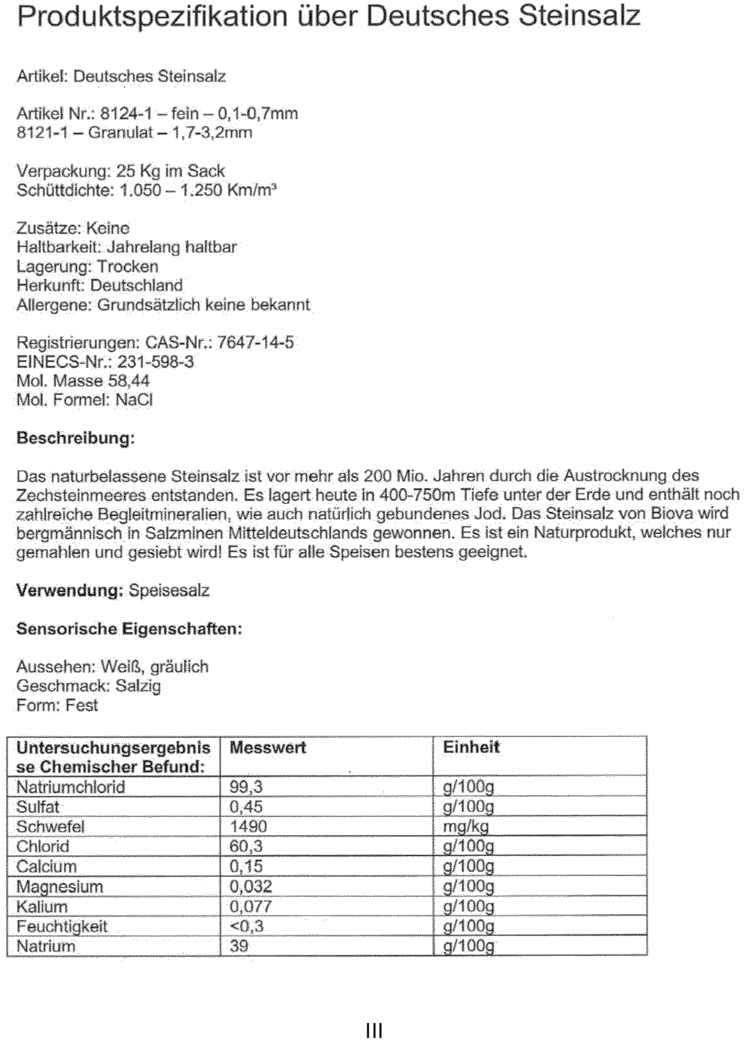

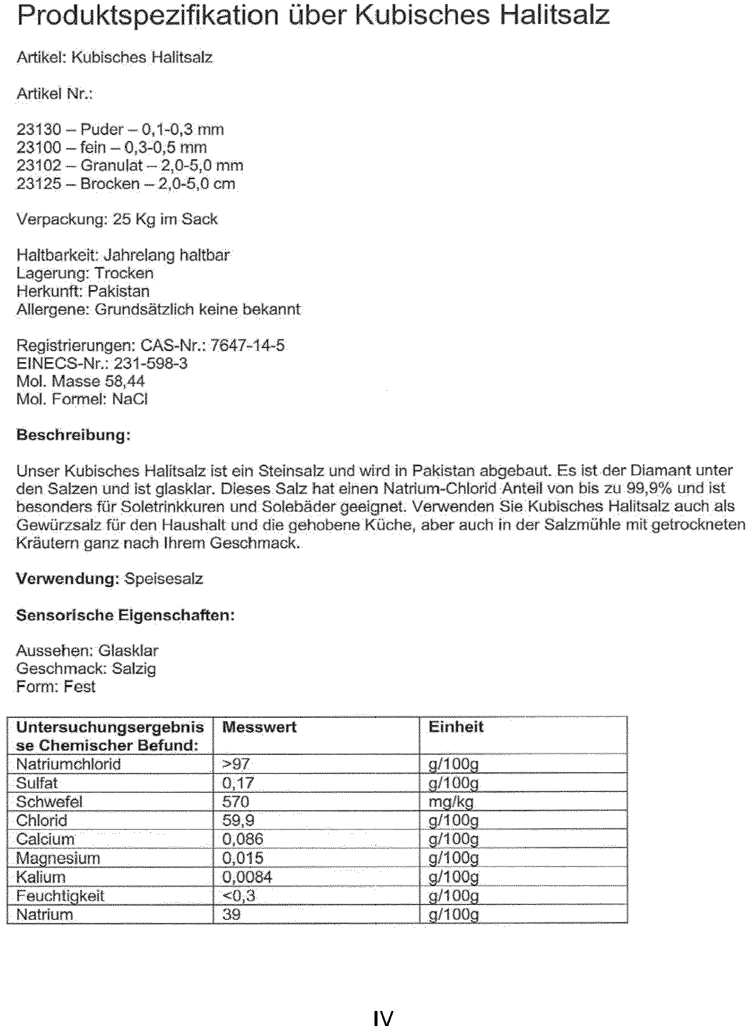

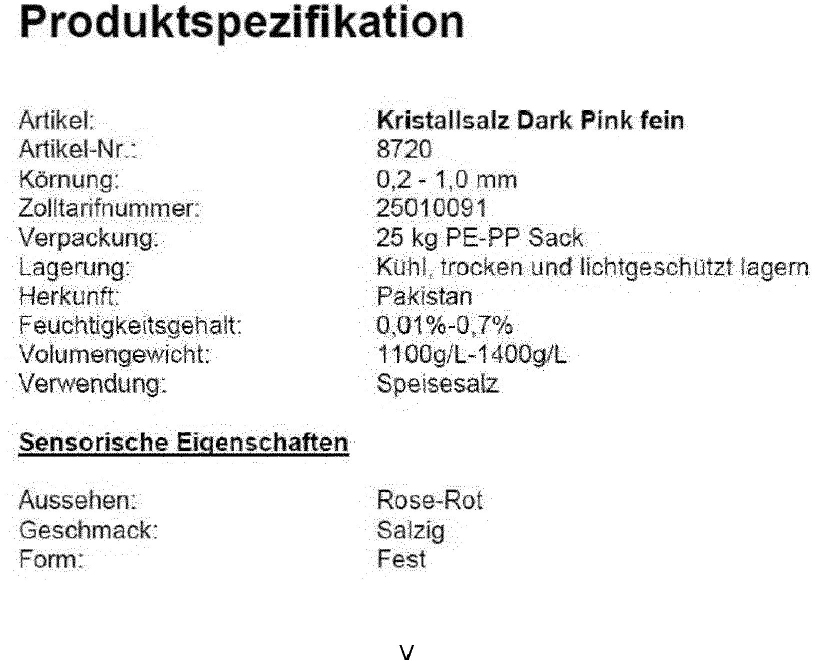

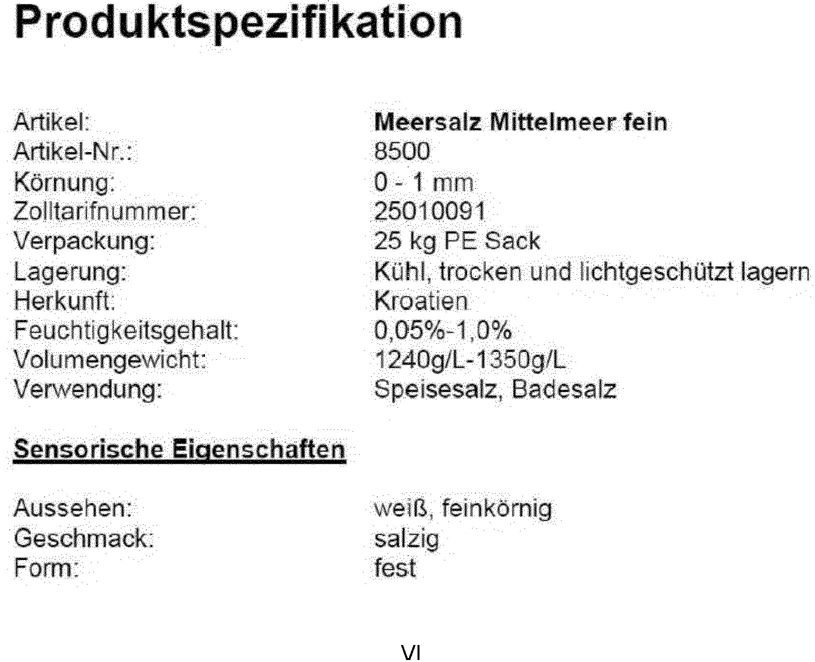

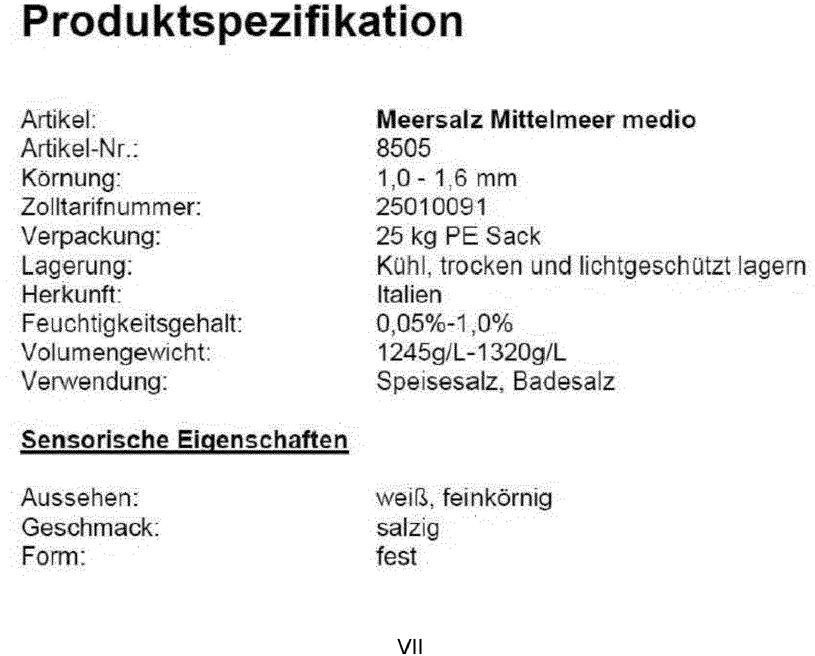

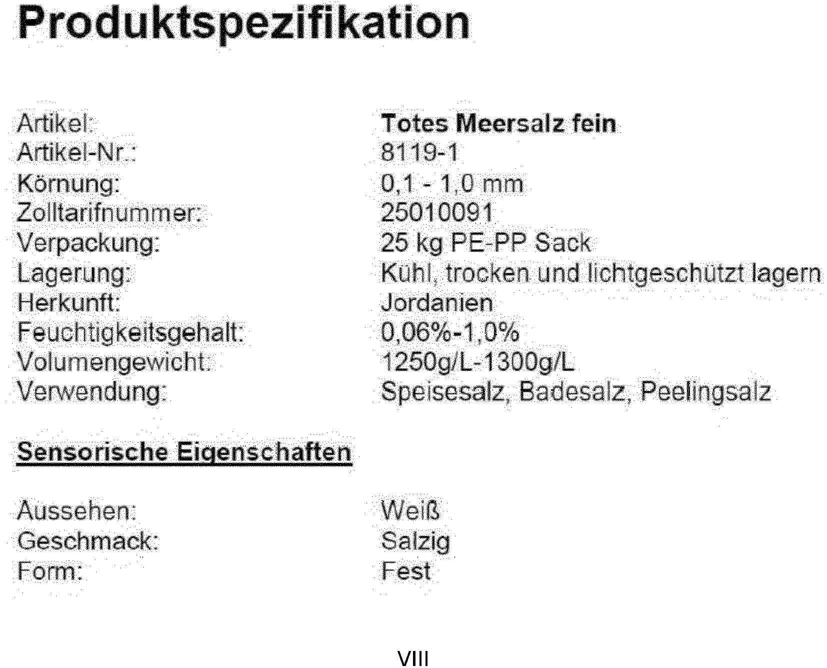

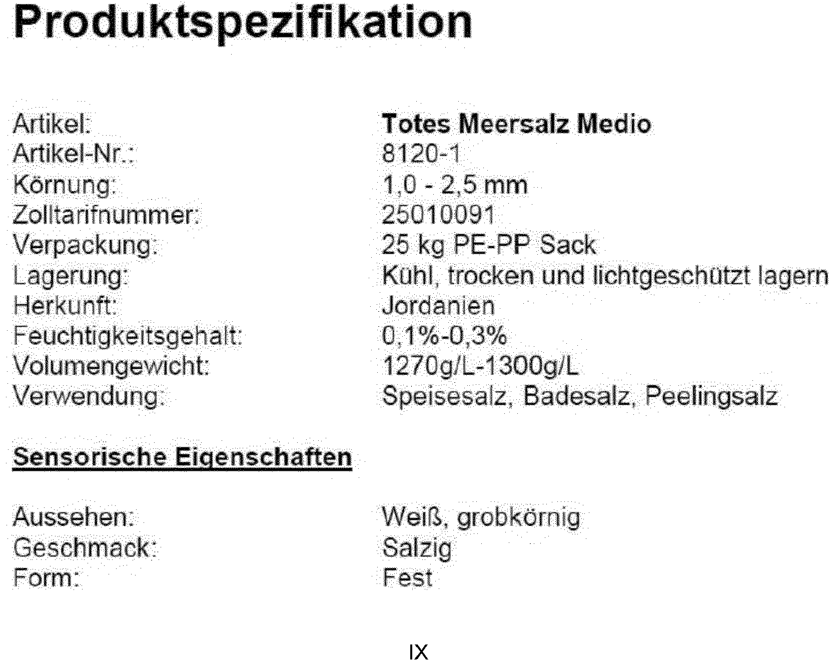

**Bezugszeichen**

| | |
|---|---|
| 1, 1', 1" | Formkörper |
| 2 | Applikator |
| 3 | Armatur |
| 4 | Schlauchleitung |
| 5 | Aufnahmekammer |
| 6 | Bypass |
| 7 | Mehrwegeventil/Bedienheber |
| 8 | (Zufluss)eingang |
| 9 | (Zufluss)ausgang |
| 10 | Einlassseite |
| 11 | Auslassseite |
| 12 | Muffe/Schraube |
| 13 | Acrylrohr/Glasrohr |
| 14 | Edelstahlrohr |
| 15 | Rückflussverhinderer |
| 16 | Gewindemuffe |
| 17 | Einklebemuffe |
| 18 | Rückflussverhinderer |
| 19 | Deckelaufnahme/Bypassanschluss |
| 20 | Sieb/Verschluss |
| 21 | Deckel |
| 22 | Abgang |
| 23 | Bohrung |
| 24 | Sieb(körper) |
| 25 | Deckel |
| 26 | Siebdeckel |
| 27 | Duschkopf |
| 28 | Duscharmatur |
| 29 | Wand |
| 30 | Schlauch |
| 31 | Griffmulde |
| 32 | Einheit mit z.B. thermoelektrischen Generator |
| 33 | Monitor/Bildausgabequelle |
| 34 | Überwurfmutter zum Duschschlauch |

## Patentansprüche

1. Gepresster Formkörper (1), der aus wasserlöslichen Salzpartikeln besteht, die eine vorbestimmte Partikelgröße aufweisen, wobei der Mehrzahl der gepressten Partikel eine Partikelgröße aufweist, die im Bereich von 0,2mm bis 3,5mm liegt, vorzugsweise im Bereich von 0,5mm bis 2,5mm liegt, wobei die Partikel mit einem vorbestimmten Pressdruck zur Ausbildung des Formkörpers verpresst sind, wobei der Formkörper eine vorbestimmte Form, Oberfläche und Querschnitt, ein vorbestimmtes Volumen und eine vorbestimmte Masse aufweist und innerhalb einer vorbestimmten Zeit, vorzugsweise innerhalb von 1 bis 10 Minuten, vorzugsweise etwa 1 bis 5 Minuten durch eine vorbestimmte Mindestmenge Wasser, die innerhalb der vorbestimmten Zeit den Formkörper umfließt, die Partikel des Formkörpers zu einen vorbestimmten Prozentsatz, vorzugsweise mehr als 90%, besonders bevorzugt bis zu 100% auflöst.

2. Formkörper (1) nach Anspruch 1, **dadurch gekennzeichnet, dass** das im Formkörper (1) befindliche Salz eine vorbestimmte Zusammensetzung aufweist,
und/oder
**dadurch gekennzeichnet, dass** die Partikel, aus denen der Formkörper (1) zusammengesetzt ist, mit einem spezifischen Pressdruck verpresst sind, wobei dieser Pressdruck größer ist als 10 KN/cm², vorzugsweise etwa 18 KN/cm² beträgt, aber kleiner ist als ein Höchstpressdruck, z.B. kleiner ist als 50 KN/cm², bevorzugt kleiner ist als 25 KN/cm².

3. Formkörper (1) nach einem der vorhergehenden Ansprüche, wobei der Formkörper (1) eine vorbestimmte Länge (x), Breite (y) und Höhe (Tiefe) (z) aufweist, wobei die Länge des Formkörpers im Bereich von 30 bis 250 mm liegt, vorzugsweise im Bereich von 50 bis 150 mm liegt, die Breite des Formkörpers im Bereich von 5 bis 50 mm liegt, vorzugsweise im Bereich von 15 bis 30 mm liegt und die Höhe des Formkörpers im Bereich von 5 bis 50 mm liegt, vorzugsweise im Bereich von 15 bis 30 mm liegt.

4. Formkörper (1) nach einem der vorhergehenden Ansprüche, **dadurch gekennzeichnet, dass** der Formkörper (1) einen Querschnitt aufweist, der spiegelsymmetrisch ist aber vorzugsweise nicht rotationssymmetrisch, bevorzugt oder der Formkörper (1) einen Querschnitt aufweist, der im Querschnitt unsymmetrisch ist,
und/oder
der Formkörper (1) einen Querschnitt aufweist, dessen größter Durchmesser vorzugsweise kleiner als 21 mm ist, vorzugsweise kleiner ist als 16 mm.

5. Formkörper (1) nach einem der vorhergehenden Ansprüche, **dadurch gekennzeichnet, dass** bis zu einem bestimmten Prozentsatz, vorzugsweise zwischen 1 und 25 %, vorzugsweise 10 bis 25 % oder darunter, z. B. 5% oder weniger Additive den Partikeln des Formkörpers beigemischt sind, vorzugsweise Zusätze, die eine körnige Struktur aufweisen, die vorzugsweise in der Größenordnung (oder geringer) der Partikel des Formkörpers liegen die ätherischen Öle bilden und/oder aus Pflanzenmaterial bestehen, z.B. Blätter, Stängel, Blüten oder dergleichen von Kräutern, Pflanzen, insbesondere v-+on solchen Kräutern, Pflanzen mit einer (nachgewiesenen) nachgemessenen Pflege- oder Gesundheitsfunktion,
wobei vorzugsweise das Additiv ein ätherisches Öl ist, welches vor Verpressung mit den Partikeln vermischt wird und die Partikel im Formkörper bindet, wobei das Additiv vor oder nach Verpressung des Formkörpers mit diesem vermischt wird bzw. diesem Formkörper zugesetzt wird.

6. Applikator (2) mit einer Aufnahmekammer zur Aufnahme des Formkörpers nach einem der vorhergehenden Ansprüche und/oder eines Siebs mit darin untergebrachten Zusätzen, die sich wenigstens teilweise im Wasser auflösen lassen, insbesondere Kräuter, ätherische Öle oder dergleichen, wobei die Aufnahmekammer für eine vorbestimmte Querschnittsform, z.B. kreiszylindrisch oder dergleichen, und einen vorbestimmten Durchmesser aufweist, der um ein lichtes Maß größer ist als der größte Durchmesser des Formkörpers und/oder des Siebs, wobei die Aufnahmekammer eine Länge (L) aufweist, die größer ist als die Länge des Formkörpers (1) und/oder des Siebs und die Aufnahmekammer eine verschließbare Öffnung zum Einsatz eines Formkörpers und/oder des Siebs in die Aufnahmekammer (5) aufweist,
wobei vorzugsweise der Applikator (2) einen (Zufluss)eingang (8) und einen (Abfluss)ausgang (9) und ein Zwei- oder Mehrwegeventil (7) aufweist, wobei das Ventil (7) in einer ersten Stellung A den Durchfluss von Wasser vom Eingang (8) des Applikators zum Ausgang (9) des Applikators (2) ermöglicht, ohne das Wasser in die Aufnahmekammer gelangt.

7. Applikator (2) nach einem der vorhergehenden Ansprüche, **dadurch gekennzeichnet, dass** das Ventil (7) bei einer zweiten Stellung B ermöglicht, dass Wasser vom Eingang (8) des Applikators (2) durch die Aufnahmekammer (5) fließt und von dort zum Ausgang (9) des Applikators (2) fließt,
und/oder
**dadurch gekennzeichnet, dass** die Aufnahmekammer (5) eine Längsausrichtung A-A aufweist, welche im Wesentlichen senkrecht zur Ausrichtung einer Achse B-B steht, die durch den Eingang und den Ausgang des Applikators gebildet wird und das die Aufnahmekammer im verbauten Zustand des Applikator im Wesentlichen horizontal oder vertikal ausgerichtet ist, sodass bei horizontaler Ausrichtung der Aufnahmekammer, diese durch horizontales Einschieben des Formkörpers mit diesem gefüllt werden kann und bei einer vertikalen Ausrichtung der Aufnahmekammer das Einschieben des gepressten Formkörpers ebenfalls aus einer vertikalen Richtung erfolgt.

8. Applikator (2) nach einem der vorhergehenden Ansprüche, **dadurch gekennzeichnet, dass** am Eingang (8) des Applikators (2) eine Schraubvorrichtung mit Innengewinde vorgesehen ist, das Standardabmaße aufweist, insbesondere ein Zollinnenmaß aufweist, nämlich ½ Zoll, ¾ Zoll oder 1 Zoll,
und/oder
**dadurch gekennzeichnet, dass** am Ausgang (9) des Applikators (2) ein Außengewinde vorgesehen ist, welches zollmaßig ist und vorzugsweise ½ Zoll, ¾ Zoll oder 1 Zoll groß ist.

9. Applikator (2) nach einem der vorhergehenden Ansprüche, **dadurch gekennzeichnet, dass** die Aufnahmekammer (5) zur Aufnahme des Formstücks (1) aus einem transparentem Wandmaterial (13) besteht, vorzugsweise aus Glas, Acrylmaterial, Polyvinylchlorid (PVC), Polycarbon (PC), Polycarbonat (PC) oder dergleichen, wobei die Aufnahmekammer (5) eine Einlassseite (10) und eine Auslassseite (11) aufweist, wobei die Aufnahmekammer an seiner Einlassseite (10) an einem entsprechenden Gegenstück (14) des Applikators (2) befestigt ist und das die Aufnahmekammer (5) an ihrer Auslassseite (11) einen Leitungsanschluss z.B. Anschluss für einen Bypass (6) aufweist, welcher in den Auslass (9) des Applikators (2) mündet,
wobei vorzugsweise die Aufnahmekammer (5) an ihrem unteren (freien) Ende einen Verschluss aufweist, z.B. einen Excenterverschluss, Schiebekeilverschluss, Schraubverschluss, Bajonettverschluss, Nutfederverschluss, Stopfen oder dergleichen und bei Ausbildung eines Schraubverschlusses die Aufnahmekammer an ihrem unterem Ende ein Gewinde aufweist, auf welches eine Schraube (12) mit einem entsprechenden Gewinde und eine Dichtung zum wasserdichten Verschließen der Aufnahmekammer aufschraubbar ist.

10. Applikator (2) nach einem der vorhergehenden Ansprüche, **dadurch gekennzeichnet, dass** bei Einsatz eines Formkörpers (1) im Innenraum der Aufnahmekammer (5) zwischen der Außenwandung des Formkörpers einerseits und der Innenwandung der Aufnahmekammer (5) sich wenigstens ein Fließkanal bildet, durch welchen Wasser, welches von der Einlassseite (10) der Aufnahmekammer (5) zur Auslassseite (11) der Aufnahmekammer (5) am Formkörper (1) vorbeifließen kann,
wobei vorzugsweise zwischen der Innenwandung des Aufnahmeraums und dem Außenumfang des Formkörpers wenigstens 4 Fließkanäle vorzugsweise 5 oder mehr Fließkanäle gebildet werden.

11. System zur Anreicherung von Wasser mit einer gewünschten, vorbestimmten Salzkonzentration, wobei das System einen Applikator (2) nach einem der vorhergehenden Ansprüche umfasst, in den ein fester Formkörper (1) nach einem der vorhergehenden Ansprüche eingesetzt wird bzw. einsetzbar ist.

12. Mischbatterie mit einem Applikator nach einem der vorhergehenden Ansprüche, wobei der Applikator ein integraler Teil der Mischbatterie ist und die Mischbatterie und der Applikator ein einziges Teil bilden und mit einem Werkzeug nicht zerstörungsfrei voneinander trennbar sind.

13. Kombination eines Duschkopfs und eines Applikators nach einem der vorhergehenden Ansprüche, wobei der Applikator ein integraler Teil des Duschkopfes ist und mit diesem ein Teil bildet und der Applikator und Duschkopf mit einem Werkzeug nicht zerstörungsfrei voneinander lösbar sind, wobei der Duschkopf einen Handgriff aufweist und der Applikator den Handgriff des Duschkopfs bildet oder in diesem untergebracht ist.

14. Kombination des Duschkopfs eines Applikators nach einem dervorhergehenden Ansprüche, wobei der Duschkopf und der Applikator lösbar miteinander verbunden sind, vorzugsweise über eine Hauptverbindung und wobei der Applikator den Handgriff des Duschkopfs bildet.

15. Duscheinrichtung mit einem Applikator nach einem der vorhergehenden Ansprüche, bei der wenigstens ein Sensor ausgebildet ist zur Messung eines vorbestimmten Parameters, z.B. der Wassertemperatur, der Duschstartzeit, des Wasserdrucks, der Duschendzeiten, der Duschdauer, des Wasserflusses während des Duschens, des Salzgehalt im Wasser, etc., wobei die von dem oder den Sensoren erfassten Daten über eine optional ausgebildete Standardkommunikationsschnittstelle, z.B. WLAN, Bluetooth, Ethernet, etc. an eine Datenverarbeitungseinrichtung, z.B. Smartphone, Tablet, Computer, PC, etc. übertragen werden können und zur Energieversorgung der Sensoren, wie auch zur Datenübertragung eine Batterie, ein Akkumulator, ein Kondensator und/oder ein thermoelektrischer Generator ausgebildet ist, wobei die Sensoren, Hardware der Kommunikationsschnittstelle wie auch die Energieerzeugung vorzugsweise als ein integrales Bauteil ausgebildet und im oder am Duschkopf oder Duschschlauch (der am Duschkopf und/oder Applikator anschließbar ist) oder im oder am Applikator untergebracht sind,
wobei vorzugsweise die Duscheinrichtung eine Anzeigeeinrichtung zur Anzeige eine oder mehrere Duschparameter aufweist, z.B. zur Anzeige der Wassertemperatur, der Duschstartzeit, des Wasserdrucks, der Duschendzeit, der Duschdauer, des Wasserflusses während des Duschens, des Salzgehalts im Wasser, etc. oder dergleichen und die Anzeigeeinrichtung mit wenigstens einem Sensor gekoppelt oder verbunden ist, um gemessene Sensordaten des Duschbetriebs anzuzeigen, z.B. die Temperatur des Duschwassers, Salzgehalt des Duschwassers, Duschdauer oder dergleichen.
